# EUROPEAN PATENT APPLICATION

(11) **EP 2 322 149 A1**
(43) Date of publication of application: **18.05.2011**
(21) Application number: 09382239.3
(22) Date of filing: 03.11.2009
(51) Int. Cl.: A61K 31/00, A61K 31/47, A61K 31/4709, A61K 31/60, A61K 31/78, A61P 25/00

(54) **Methods and compositions for the treatment of ischemia**

(71) Applicant: Universidad del Pais Vasco, 48940 Leioa Vizcaya (ES)
(72) Inventor: Domercq García, María, E-48940, Leioa - Vizcaya (ES); Matute Almau, Carlos, E-48940, Leioa - Vizcaya (ES)
(74) Representative: ABG Patentes, S.L.

(57) **Abstract**

The invention relates to panncxin hemichannel inhibitors and P2X inhibitors for use in the treatment of central nervous system ischemia, more precisely, white matter ischemia.

SEQUENCE LISTING

<110>  Universidad del País Vasco
<120>  METHODS AND COMPOSITIONS FOR THE TREATMENT OF ISCHEMIA

<130>  P5358EP00

<160>  20

<170>  PatentIn version 3.5

<210>  1
<211>  2782
<212>  DNA
<213>  Homo sapiens

<400>  1

gggcggcgcg gaggggcagg gccagaggga agcgctttgt tccgcgcgtg gttcccgcgc      60

ctgggggtgc gcgggagagg cgcgaatccg agtgccgcgc gcggcccggg gacttgcacg     120

ggcgtgcggg gtggaaccgc aggaagcgga gctctcgggt tcccgccccg ccccgccccg     180

ccggcggcgg aggcagcgag cgcgagagcc cagcggagtc gctgggagcc tgaggcaccg     240

agacacaaag gcaggcggga tgcgggagca ggcaaaggga aagcgaaagc cgcgcgcccg     300

gccggtgact gggtgaaggc gccgcgcagc tttcccgacg ccggctgtac ccggacctcc     360

tggtcgagcc tggcgcgccg cagccatggc catcgctcaa ctggccacgg agtacgtgtt     420

ctcggatttc ttgctgaagg agcccacgga gcccaagttc aaggggctgc gactggagct     480

ggctgtggac aagatggtca cgtgcattgc ggtggggctg cccctgctgc tcatctcgct     540

ggccttcgcg caggagatct cgattggtac acagataagc tgtttctctc caagttcttt     600

ctcctggcgt caggctgcct ttgtggattc atattgctgg gcggctgttc agcagaagaa     660

ctcactgcag agcgagtctg gaaacctccc actgtggctg cataagtttt tcccctacat     720

cctgctgctc tttgcgatcc tcctgtacct gcccccgctg ttctggcgtt tcgcagctgc     780

tcctcatatt tgctcagact tgaagtttat catggaagaa cttgacaaag tttacaaccg     840

tgcaattaag gctgcaaaga gtgcgcgtga ccttgacatg agagatggag cctgctcagt     900

tccaggtgtt accgagaact tagggcaaag tttgtgggag gtatctgaaa gccacttcaa     960

gtacccaatt gtggagcagt acttgaagac aaagaaaaat tctaataatt taatcatcaa    1020

gtacattagc tgccgcctgc tgacactcat cattatactg ttagcgtgta tctacctggg    1080

ctattacttc agcctctcct cactctcaga cgagtttgtg tgcagcatca aatcagggat    1140

cctgagaaac gacagcaccg tgcccgatca gtttcagtgc aaactcattg ccgtgggcat    1200

cttccagttg ctcagtgtca ttaaccttgt ggtttatgtc ctgctggctc ccgtggttgt    1260

ctacacgctg tttgttccat tccgacagaa gacagatgtt ctcaaagtgt acgaaatcct 1320

ccccactttt gatgttctgc atttcaaatc tgaagggtac aacgatttga gcctctacaa 1380

tctcttcttg gaggaaaata taagtgaggt caagtcatac aagtgtctta aggtactgga 1440

gaatattaag agcagtggtc aggggatcga cccaatgcta ctcctgacaa accttggcat 1500

gatcaagatg gatgttgttg atggcaaaac tcccatgtct gcagagatga gagaggagca 1560

ggggaaccag acggcagagc tccaaggtat gaacatagac agtgaaacta aagcaaataa 1620

tggagagaag aatgcccgac agagacttct ggattcttct tgctgatgat ttttttcctt 1680

gagctgtaaa tctgtgactt ctgcgacatg ggatttaatt tggctaaagc acccctgttg 1740

gtttcacagc tggtttgcaa taaatggttc ttggtggaga tactgagcat gtcttattga 1800

gtccctaatg gaaatggtga tcaacaaaag gttatggaag aatggtttat gaacttccca 1860

taggaagcac ctgagagata gtaaactgca gcaagtaact atgtgtaagt cctcatcaaa 1920

tgaaaagcag aaagacaaga acaattagtc aagagcagta gccctgtcag agcctcggag 1980

caataccttt ctgtacccgt ggtgagacaa gacccagagc tactggaaaa caagcacttt 2040

ggaagatttg ttttgttttc atggaataat aatatgtcag ggtataattt aacgtgagtt 2100

tcttatgtgc ccttaaagac tgttagacaa gaaaagcatt cactggctaa taatccatag 2160

gtcgacctat gtcctaagtt aggtgtaagg tccgatgcct tggcccacac tcgagctctc 2220

tttacattgt tagttgtcaa ccttggctga tggaaatccc gtaaccacta tttgttgcac 2280

tgtgccttga agggcagcag gcccaagtgc tgctctgact gaaaactgag ttaacaagat 2340

gaaatctaaa ggatattcac agtgacttca attcaggaag aatgcttcca aaagagccca 2400

gtggggaaat ctgacatcac agaagacatt aattcagtca ctttcaaaga gtttgtctac 2460

aggcggtttc tctgttatca aaggcatttg aaataggatt ttacttaaac aataatggaa 2520

cacaggagta tttaaagtga agaacacttt gcctgaatgt gatcagggca cataagtgac 2580

attggcatgc ttcatatggc gtgcttggag ccagaaaaac ttagcggttt attttgttta 2640

tatttaagca cagctttaaa aaattcatta tcgtttattc agtgtccgaa ttgaggccat 2700

ttgggaagaa aattctagca ctggtggaga attatagaat aaagattata aatggttgga 2760

taagacaaaa aaaaaaaaaa aa 2782

<210> 2
<211> 1609
<212> DNA
<213> Homo sapiens

<400> 2

atgtcacttg cacacacagc tgcagagtac atgctctcag atgccctgct gcctgaccgc 60

aggggacccc gcctcaaagg actgcgtctg gaactgcccc tggaccggat agtcaagttc 120

gtagctgtgg gctccccctt gttgctgatg tccctggcat tcgcccagga gttctcctct 180

gggtctccga tcagctgctt ctctcccagt aacttcagca tccggcaggc agcctacgtg 240

gacagctcct gctgggactc actgcttcac cataagcagg acgggcctgg ccaggacaaa 300

atgaaatctc tctggcccca caaggccctc ccctactccc tgctggccct ggccttgctc 360

atgtacctgc cggtgctgct gtggcagtat gcagctgtgc cagccctcag ctccgatctg 420

ctgttcatca tcagcgaact ggacaaatct tataatcgct ccatccgcct cgtgcagcac 480

atgctgaaga tccggcagaa gagttccgac ccctatgtgt tctggaatga gctggagaag 540

gctcggaaag aacgatactt tgaattccct ttgctagagc ggtacctggc atgtaagcag 600

cgttcacatt cgctagtggc tacctacctc ctgaggaact ccctcttgct catcttcacc 660

tccgccactt acctatacct tggtcatttc catctggatg tcttcttcca ggaagaattc 720

agctgctcca tcaagacagg gctgctaagt gatgagaccc atgtccccaa tctgatcaca 780

tgcaggctga catcactgtc cattttccag attgttagcc tctccagtgt agcaatatac 840

accatattgg ttccagtgat aatatacaac ctcacacggc tatgtcggtg ggacaaacga 900

cttttatctg tctatgagat gctcccagct tttgatctcc tcagcagaaa gatgctagga 960

tgtcccatca atgacctcaa tgtgatcctt cttttcctcc gagctaacat ctctgagctc 1020

atctctttta gctggctgag tgtcttatgt gtgttgaagg atacaaccac ccagaagcac 1080

aatattgaca cagtagttga ttttatgact ttattggctg gcttagaacc ctcaaaaccc 1140

aaacacctca ccaactcggc atgtgatgaa cacccatagt taagaaacca tggagcaaga 1200

aagcttgtgg aaagtctctc tccttcctca taagacatgc acactaatac acatacacac 1260

caaaaaatta cacattttaa aactgctaag cttggattta actgaatcat atatctttta 1320

tcatgttatc ctaaaagtga gaagacataa ccaagacatg gaaataaatg tgaaagctgg 1380

agccgaagag tcaaagagct aaaaaattaa gtctagaaca ttctatgagg atagtataaa 1440

taaaaagaaa tacagtctag acatgctgca aggaaagaag attctaaagt ccgtttatgg 1500

aggcaattcc atatcctttc ttgaacgcac attcagctta ccccagagag caagtgaggc 1560

aatctggcaa aagattaata aagatgtaaa cccctggaaa aaaaaaaaa 1609

<210> 3
<211> 3069
<212> DNA
<213> Homo sapiens

<400> 3
atgcaccacc tcctggagca gtcggcggac atggcgaccg cgctgctggc gggagagaag 60

ctgcgggagc tgatcctgcc gggcgcgcag gacgacaagg cgggcgcgct ggccgcgctg 120
cttctgcagc tgaagctgga gctgccgttc gaccgggtgg tcaccatcgg caccgtgctg 180
gtgcccatcc tgctggtcac cctggtcttc accaagaact tcgcagagga acccatttac 240
tgttacaccc cgcacaactt cacgcgcgac caggcgctgt acgcccgcgg ctactgctgg 300
acggagctgc gggacgcgct gcccggcgtg gacgccagcc tgtggccgtc gctgtttgag 360
cacaagttcc tgccctacgc gctgctggcc ttcgccgcca tcatgtacgt gcccgcgctg 420
ggctgggagt tcctggcctc cacgcgcctc acctccgagc tcaacttcct gctgcaggag 480
atcgacaact gttaccaccg ggcggccgag ggccgcgcgc ccaagatcga gaagcagatc 540
cagtccaagg gcccgggcat cacggagcgc gagaagcgcg agatcatcga gaacgcggag 600
aaggagaaga gcccggagca gaacctgttc gagaagtacc tggagcgccg cggccgcagc 660
aacttcctgg ccaagctgta cctggcgcgg cacgtgctga tcctgctgct gagcgccgtg 720
cccatctcct acctgtgcac ctactacgcc acgcagaagc agaacgagtt cacctgcgcg 780
ctgggcgcgt ccccggacgg ggcggcaggt gcggggcccg cggtgcgcgt gagctgcaag 840
ctcccgtccg tgcaactgca gcgcatcatc gcgggcgtgg acatcgtgct gctgtgcgtc 900
atgaacctca tcatcctcgt caacctcatc cacctcttca tcttccgcaa gagcaacttc 960
atcttcgaca agctgcacaa ggtgggcatc aagacgcgcc ggcagtggcg ccgctcgcag 1020
ttctgcgaca tcaacatcct ggccatgttc tgcaacgaga accgcgacca catcaagtcg 1080
ctcaaccggc tggacttcat caccaacgag agcgacctca tgtacgacaa cgtggtccgg 1140
cagctgctgg cggcgctggc gcagtccaac cacgacgcca cccccacggt gcgcgactcg 1200
ggggtgcaga ccgtggaccc cagcgccaac cccgccgagc ccgacggcgc cgccgagccg 1260
cccgtggtca agcggccgcg caagaagatg aagtggatcc ccaccagcaa cccgcttccg 1320
cagcccttca aggagccgct ggccatcatg cgcgtggaga acagcaaggc ggagaagccg 1380
aagcccgcgc gcaggaagac ggccacggac acgctgatcg cgccgctgct ggaccgctcc 1440
gcccaccact acaagggcgg agggggcgac ccgggccccg gccccgcccc tgcccccgcc 1500
ccgccgcccg cccctgacaa gaagcacgcg cgccacttct ccctggacgt gcacccctac 1560
atcctcggca ccaagaaggc caaggccgag gcggtgcccg ccgccctgcc cgcctcccgg 1620
agccaggagg ggggcttcct gtcccaggcg gaggactgtg ggctaggcct ggccccggcg 1680
cccatcaaag atgctccgct ccccgagaag gaaatcccgt accccacaga gccagcccgg 1740
gcagggcttc cctcgggggg cccgttccac gtccgctcac ctcccgccgc ccctgctgtg 1800
gcccctctga caccagccag cctgggcaag gcggagcccc tcaccatcct gagccgaaac 1860
gccacacacc cgctgctgca catcaacacg ctgtacgagg cccgggagga ggaggacggg 1920
ggcccccgcc tgccgcagga cgtgggggac ctcatcgcca tccctgcccc acagcagatc 1980

ctcatcgcca ccttcgacga gccgagaacg gtcgtgagta ctgtggagtt ttgagggatg     2040

gcaccgtcca ggccgccgag agcccctctg cctgtgtcgt gtggcctggc cagcctcccg     2100

gtggacacca gccctgcgtg gacgtggcct gtgcttcgcc cgcactgcgc gcatccccaa     2160

cctctgtccg catgcctggg gccttcgccc ccacgtgctc gacaggggaa cccgcccgga     2220

cggcatcgcc aggcactggc tggggtgggg aaaggtggcc cagtggagcc ggtggccagg     2280

aaggctgaag cccgcttccc atgctcctgc atcaggtgcc cagccgtggg tgggggccct     2340

gaggtgaaga gtttattttt ttagtccgtt tcgtcctggc cccgggctgt ggcgagacag     2400

cccaactccc ccagcccagc tcccccagcc cagagccagg gaagaggaag gtggggccag     2460

tcccaccagt ggggtggcca cgcccatggg gtcacatgct caggggtcac cccctgcagg     2520

gacctgatgc cctcgggtgg gagggaccga ggtccaccct cgggtcaaag gtcaacgtgc     2580

actttctcct tgtcgcctga cagacatttt attttactaa gactgctgta ccgaacaagc     2640

atatttatca tcaggagaca ggatgggttt aaagcaggat ggtgtgtgtg tgaacgggca     2700

tgagcagagg tgagcgtgag cgagcgggtg tgtatgtacg agtgtgcacg tgtgtgcgtg     2760

tgcacagagg gtgtggtgcc agcttgagtg ggagtgtgtg agtgtgagca ggcgggcgag     2820

tgcgtgagtg cacgccagcg cgtggcccat gtatgaggag tgaaggggcc caacgcaata     2880

accacgtccc ccacccgggc cccccgccgc ggctgaggcc acatggcttc ctgtgggagc     2940

cccggccggc acccggctgg tcccacccca aatacctcag ccatggagac catgtcatgc     3000

agaattaaca aggtagcacc gagcatatca ataaatatta ttctgataat caaaaaaaaa     3060

aaaaaaaaa                                                             3069

<210>  4
<211>  19
<212>  RNA
<213>  Artificial Sequence

<220>
<223>  Target sequence for the human pannexin1 siRNA 1

<400>  4
ggcugccuuu guggauuca                                                    19

<210>  5
<211>  19
<212>  RNA
<213>  Artificial Sequence

<220>
<223> Target sequence for the human pannexin1 siRNA 2

<400>  5
ggagaucucg auugguaca                                                    19

<210>  6
<211>  19
<212>  RNA
<213>  Artificial Sequence

<220>
<223> Target sequence for mouse pannexin1 siRNA 1

<400>  6
ggcugccuuu guggauuca                                                    19

<210>  7
<211>  19
<212>  RNA
<213>  Artificial Sequence

<220>
<223> Target sequence for mouse pannexin1 siRNA 2

<400>  7
ggaacuugac aaagucuac                                                    19

<210>  8
<211>  19
<212>  RNA
<213>  Artificial Sequence

<220>
<223> Target sequence for mouse pannexin1 siRNA 3

<400>  8
ggugcuggag aacauuaaa                                                    19

<210>  9
<211>  10
<212>  PRT
<213> Artificial Sequence

<220>
<223> Inhibiting peptide specific for a pannexin hemichannel 1

<400>  9

Trp Arg Gln Ala Ala Phe Val Asp Ser Tyr
1               5                   10

<210>  10

<211>  11
<212>  PRT
<213>  Artificial Sequence

<220>
<223> Inhibiting peptide specific for a pannexin hemichannel 2

<400>  10

Ala Gln Glu Ile Ser Ile Gly Thr Gln Ile Ser
1               5                   10


<210>  11
<211>  13
<212>  PRT
<213>  Artificial Sequence

<220>
<223> Inhibiting peptide specific for a pannexin hemichannel E1b

<400>  11

Ser Ser Phe Ser Trp Arg Gln Ala Ala Phe Val Asp Ser
1               5                   10


<210>  12
<211>  12
<212>  PRT
<213>  Artificial Sequence

<220>
<223> Inhibiting peptide specific for a pannexin hemichannel E1c

<400>  12

Ser Glu Ser Gly Asn Leu Pro Leu Trp Leu His Lys
1               5                   10


<210>  13
<211>  13
<212>  PRT
<213>  Artificial Sequence

<220>
<223>  Inhibiting peptide specific for a pannexin hemichannel E2a

<400>  13

Ser Ser Leu Ser Asp Glu Phe Val Cys Ser Ile Lys Ser
1               5                   10


<210>  14
<211>  14
<212>  PRT
<213>  Artificial Sequence

<220>
<223>  Inhibiting peptide specific for a pannexin hemichannel  E2b

<400>  14

Lys Ser Gly Ile Leu Arg Asn Asp Ser Thr Val Pro Asp Gln
1               5                   10


<210>  15
<211>  20
<212>  DNA
<213>  Artificial Sequence

<220>
<223>  Panx 1 forward

<400>  15
tgagaatgtg gggcagagtc                                                   20


<210>  16
<211>  21
<212>  DNA
<213>  Artificial Sequence

<220>
<223>  Panx1 reverse

<400>  16
cagtaccacc gcaaaggtca c                                                 21


<210>  17
<211>  21
<212>  DNA
<213>  Artificial Sequence

<220>
<223>  Panx2 forward

<400>  17
ttcaccaaga acttcgcaga g                                                 21


<210>  18
<211>  19
<212>  DNA
<213>  Artificial Sequence

<220>
<223>  Panx2 reverse

<400>  18
cagtagccac gggcgtaca                                                    19


<210>  19

<211>  23
<212>  DNA
<213>  Artificial Sequence

<220>
<223>  Panx3 forward

<400>  19
gtcaagttcg ttactgtggg ctt                                               23


<210>  20
<211>  20
<212>  DNA
<213>  Artificial Sequence

<220>
<223>  Panx3 reverse

<400>  20
agcagctgat cggagaccct                                                   20

## Description

### FIELD OF THE INVENTION

The invention relates to the field of ischemia, more precisely to compounds for the treatment of white matter ischemia.

### BACKGROUND OF THE INVENTION

Injury of central white matter is a major cause of functional disability in cerebrovascular disease. White matter is a target of hypoxic-ischemic injury throughout life, in clinical settings ranging from periventricular leukomalacia in the neonatal period, stroke and cardiac arrest in adults, to vascular dementia in the aging brain. The traditional view from animal studies is that gray matter is more vulnerable than white matter to ischemia. In part, this view may be an artifact of how ischemic brain injury has been studied. Most experimental work has used rodents, in which white matter constitutes only about 14% of total brain volume; however, in man, white matter is about 50% of brain volume. In fact, the metabolic rate of white matter is only modestly lower than that of gray matter and recent animal studies suggest that white matter can be damaged even by brief ischemia. Moreover, clinical experience teaches that human ischemic stroke is almost never confined to gray matter. White matter is damaged by infarction in the territory of small vessels which perfuse centrum semiovale and deeper fiber pathways, and it is also damaged by occlusion of the large intracranial arteries which supply superficial gray and white matter together. Disruption of central conduction pathways may cause disruption of motor and sensory function, neurobehavioral syndromes and cognitive impairment. Lacking neuronal cell bodies, dendrites, or synapses, white matter is unlikely to be injured by many of the mechanisms defined specifically in gray matter. Along with growing recognition of the importance of white matter ischemia in clinical disease, there have been recent advances in white matter pathophysiology, pharmacology, and imaging.

White matter is injured by hypoglycemia, and studies have shown that glycogen plays an important role in protecting against this injury. Only astrocytes (not neurons or oligodendrocytes) contain glycogen. In the absence of glucose, astrocyte glycogen is converted to lactate and transferred via the extracellular space to neighboring axons. Within axons, lactate is converted back to pyruvate and fuels oxidative energy production to sustain axon function and stave off injury for periods up to 30 minutes. Thus, it appears that the period of protection from hypoglycemic injury is determined by the astrocyte glycogen content; by increasing glycogen content the duration of protection is extended.

Oligodendrocytes, the myelin-forming cells of the central nervous system, are highly vulnerable to injury from energy deprivation or oxidative stress. Oligodendrocytes express functional glutamate receptors of non-NMDA types (AMPA and kainate-preferring receptors), and they can be killed by overactivation of these glutamate receptors.

Kanellopoulos GK, et al. (Stroke. 2000; 31:1945-1952) describe that AMPA/kainate antagonists reduce white matter injury in rodent models of spinal cord ischemia and McCracken E, et al. (J Cereb Blood Flow Metab. 2002; 22:1090-1097) describe that AMPA/kainate antagonists reduce white matter injury in transient focal cerebral ischemia.

However there is still the need of further molecules for the treatment of white matter ischemia.

### SUMMARY OF THE INVENTION

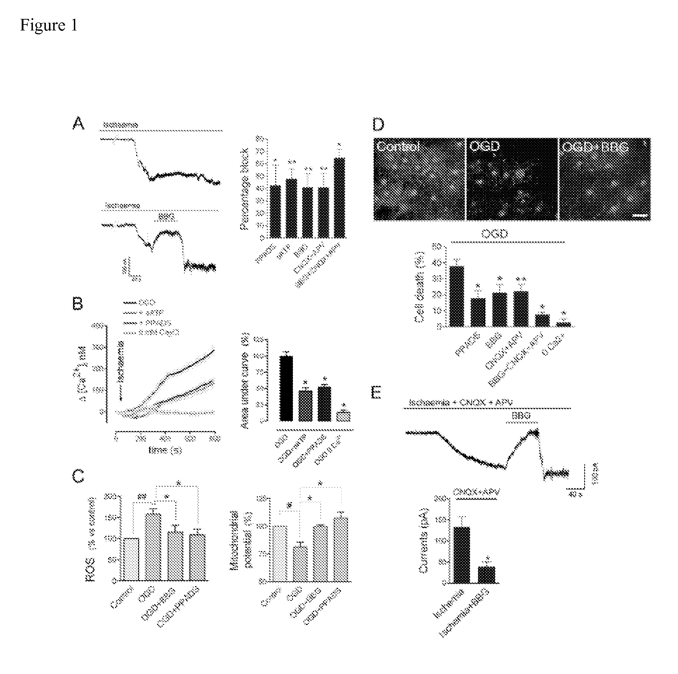

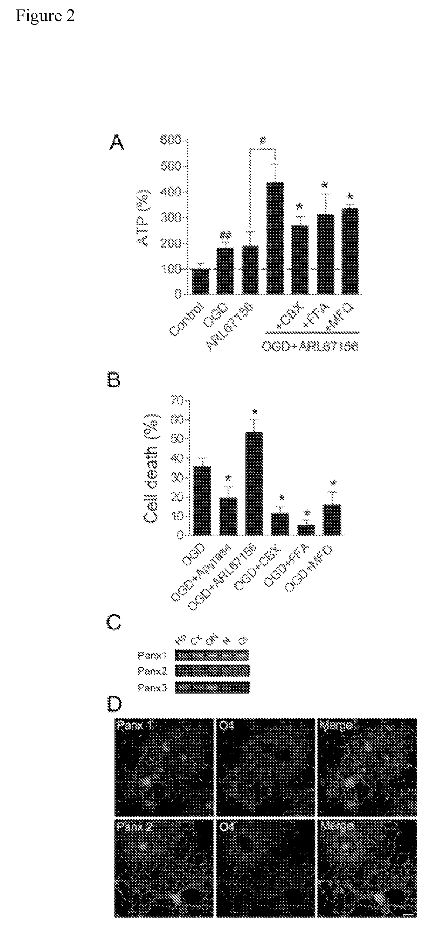

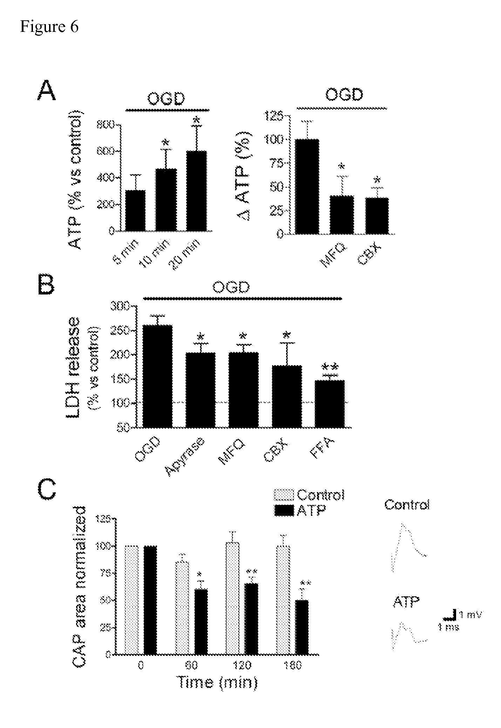

The inventors have shown that in addition to glutamate, enhanced ATP signaling during ischaemia is also deleterious to oligodendrocytes and myelin, and impairs white matter function. The inventors have seen that ischaemic oligodendrocytes in culture display an inward current and cytosolic Ca²⁺ overload, which is partially mediated by P2X7 receptors (Figure 1). The inventors have surprisingly seen that oligodendrocytes release ATP after oxygen-glucose-depletion (OGD) through the opening of pannexin hemichannels and that ischemia induced mitochondrial depolarization as well as oxidative stress culminating in cell death are partially reversed by P2X7 receptor antagonists, by the ATP degrading enzyme apyrase and by blockers of pannexin hemichannels (Figures 2 and 6). The experiments disclosed herein show how ischaemic damage in isolated optic nerves, which share the properties of brain white matter, is greatly attenuated by all said drugs (Figure 6). Ultrastructural analysis and electrophysiological recordings demonstrated that P2X7 antagonists prevent ischaemic damage to oligodendrocytes and myelin, and improved action potential recovery after ischaemia. These data indicate that ATP released during ischaemia and the subsequent activation of P2X7 receptor is critical to white matter demise during stroke and point to this receptor type and the pannexin hemichannels as a therapeutic target to limit tissue damage in cerebrovascular diseases.

Thus, in an aspect, the invention relates to a pannexine hemichannel inhibitor for the use in the treatment of central nervous system ischemia.

In another aspect, the invention relates to a method of inhibiting ATP-triggered central nervous system cell death, said method comprising contacting central nervous system cells with a pannexin blocker under conditions effective to inhibit ATPtriggered central nervous system cell death, wherein the blocker antagonist inhibits ATP flow through the pannexin hemichannel.

In another aspect, the invention relates to a P2X inhibitor for the use in the treatment of central nervous system ischemia.

### BRIEF DESCRIPTION OF THE FIGURES

**Figure 1****.** Effects of P2X7 receptor antagonists on ischaemic inward currents, Ca²⁺ increase and cell death in optic nerve oligodendrocytes. **(A)** *Left,* Voltage-clamped recording of cultured oligodendrocytes showed activation of an inward current during chemical ischaemia (top). Representative recording showing partial block of the ischaemic inward current by BBG (50 nM; bottom). *Right,* Percentage block of inward current by purinergic antagonist PPADS (100 µM), by P2X7 selective antagonists oATP (1 mM) and BBG (50 nM), by glutamate receptor antagonists CNQX (30 µM) and APV (100 µM) and by glutamate receptor antagonists plus BBG (50 nM). **(B)** Traces illustrating the time course of [Ca²⁺]_{I} increase in oligodendroglial somata after chemical ischaemia. The increase was abolished in Ca²⁺-free aCSF and significantly reduced in the presence of oATP (1 mM) and PPADS (100 µ,M).*<0.01, **^{<}0.001. (C) 1 h exposure to OGD induced the generation of radical oxygen species (ROS) and mitochondrial depolarization, that was prevented by incubating with P2X7 antagonists BBG (50 nM) and PPADS (100 µM). **(D)** Effects of oxygen and glucose deprivation (OGD) on the viability (green calcein fluorescence) of optic nerve oligodendrocytes. Scale bar = 25 µm. Cell death was prevented by removing Ca²⁺ from the extracellular medium, and reduced by incubating with PPADS (100 µM), BBG (50 nM) and CNQX (30 µM) plus APV (100 µM). Blocking both P2X7 and ionotropic glutamate receptors with BBG + CNQX + APV nearly abolished OGD-induced cell death. Symbols denote statistical significance versus control (#) or versus drug-free OGD (*). #_{,} *<0.05, ##, **<0.005. (E) Trace and histogram illustrating the effect of BBG (50 nM) on ischemic inward currents in cells in which CNQX and APV are present from the start of OGD.
**Figure 2****.** OGD induces ATP release from oligodendrocytes through pannexin hemichannels. **(A)** Luminescence measurement of ATP release after 1h OGD in cultured optic nerve oligodendrocytes. Release was potentiated in the presence of the ecto-ATPase inhibitor ARL67156 (100 µM). ATP levels after OGD (in the presence of ARL67156) were significantly reduced by pannexin hemichannel inhibitors CBX (20 µM), FFA (100 µM) and MFQ (1 nM). **(B)** Oligodendrocyte death was related to ATP levels since it was inhibited by blocking ATP release with ATP degrading enzyme apyrase (20 U/ml) and with CBX, FFA and MFQ and potentiated with ARL67156. Data are mean ± s.e.m. of 3-7 independent experiments performed in triplicate. Symbols denote statistical significance versus control (#) or versus OGD (*) *, #<0.05, **, ##<0.005. (C) RT-PCR detection of mRNA encoding pannexin 1, 2 and 3 in hippocampus (Hp), cerebral cortex (Cx), optic nerve (ON), cultured neurons (N) and cultured oligodendrocytes (01). **(D)** Pannexin 1 and 2 labelling in 04-positive oligodendrocytes. Scale bar = 15 µ pm.

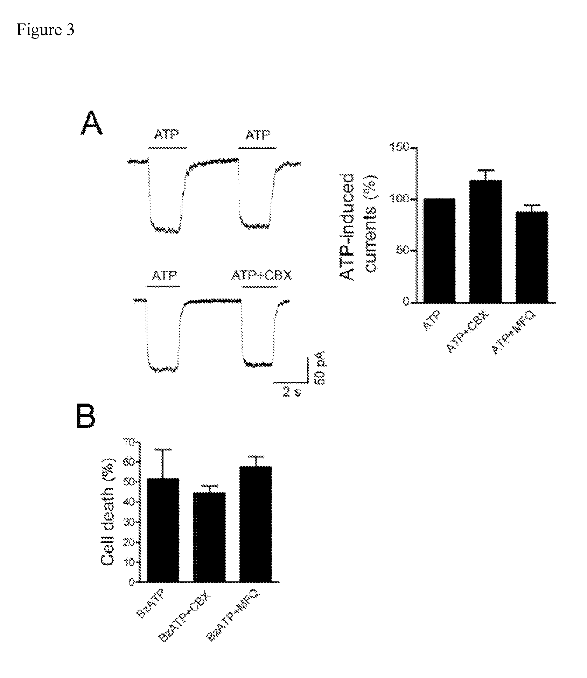

**Figure 3****.** Hemichannel inhibitors do not block P2X7 receptors in oligodendrocytes. (A) ATP (1 mM)-evoked inward currents in whole-cell clamped oligodendrocytes at -70 mV were not reduced in the presence of the hemichannel blockers carbenoxolone (CBX, 20 µM; n= 10) and mefloquine (MFQ, 1 nM; n= 6). *Left,* Representative traces showing two consecutive pulses of ATP (top; 1 mM) and a pulse of ATP (1 mM) followed by a pulse of ATP (1 mM) + CBX (20 µM) (bottom). *Right,* Bar graph summarizing the currents normalized to the first pulse of ATP (currents did not desensitize after longer exposure to agonist or after 10 consecutive pulses). **(B)** Oligodendrocyte cell death in optic nerve cultures after 15 min exposure to BzATP (100 µM) in the absence or in the presence of CBX (20 µM) and MFQ (1 nM). Cell death was measured 24h later with the calcein viability assay (see "Methods" in the Example).

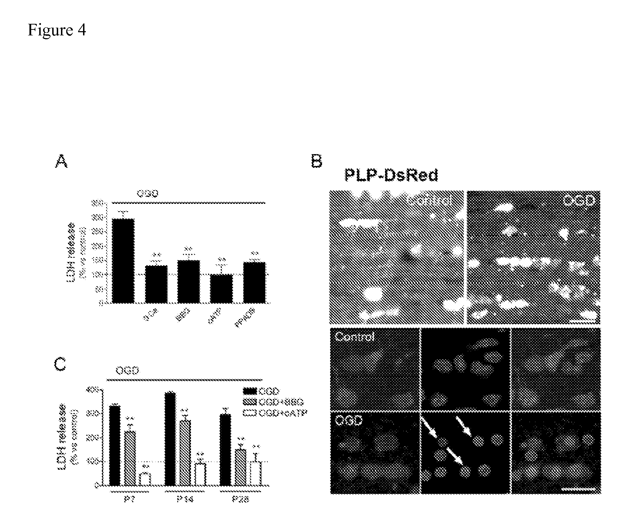

**Figure 4****.** P2X7 receptor activation mediates ischaemic damage to oligodendrocytes in the optic nerve. **(A)** Damage to oligodendrocytes after OGD, quantified by LDH release, was prevented by removing extracellular Ca²⁺ and by incubating with P2X7R antagonists BBG (50 nM), o-ATP (1 mM) and PPADS (1 mM) (n=4-13). **(B)** PLP-DsRed oligodendrocytes before (Ctrl) and after 1h OGD. *Top,* Dramatic fluorescence loss was seen in both processes and somata, indicating severe oligodendrocyte damage. *Bottom,* Hoechst labeling revealed nuclei condensation in OGD-treated optic nerves (arrows). Scale bar = 15 µm. (C) BBG and oATP decreased OGD-induced damage in premyelinating (P=7), myelinating (P=14) and mature (P=28) optic nerve (n= 4 in each group).

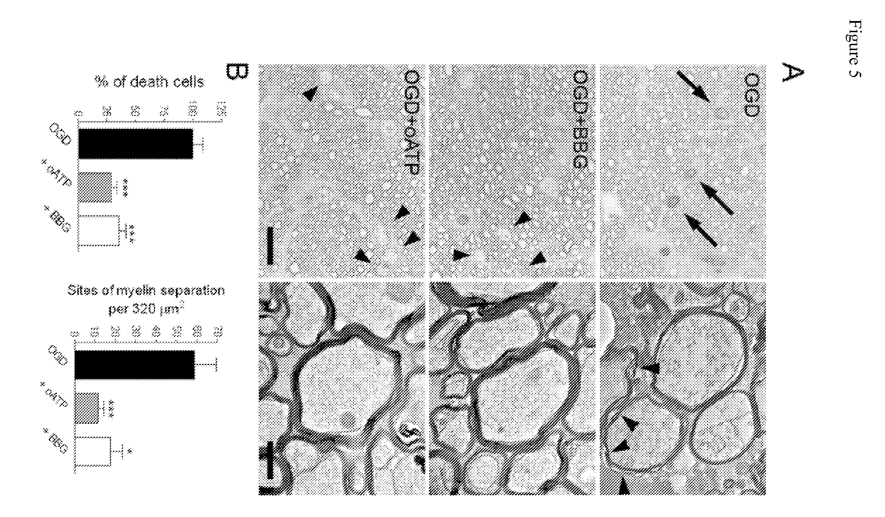

**Figure 5****.** P2X7 inhibition protects myelin against ischaemic damage. **(A)** High resolution analysis after 1h OGD showed the presence of numerous pyknotic oligodendrocytes (arrows in left) and severe damage to myelin, with separation of the compact lamellae (arrowheads in right). In the presence of BBG (50 nM) and oATP (1 mM), the number of pyknotic oligodendrocytes and the extent of myelin damage after OGD were greatly diminished. Note that most oligodendrocytes showed non condensed nuclei (arrowheads in left) in BBG and o-ATP treated optic nerves. Scale bar, 20 µm and 1 µm in left and right respectively. **(B)** Graph summarizing the effect of BBG and oATP in oligodendroglial pyknosis and myelin damage. *p<0.05, * * *p<0.0001
**Figure 6****.** Optic nerve releases ATP after OGD, which induces conduction failure. (A) *Left,* luminescence detection of ATP release from optic nerve after OGD. Maximal ATP release was detected after 20 min OGD. *Right,* ATP release was reduced by pannexin blockers MFQ (1 µM), CBX (100 µM) (n= 4). **(B)** OGD damage was reduced in the presence of apyrase (20 U/ml) and pannexin blockers MFQ (1 µM), CBX (100 µM) and FFA (1 mM) (n= 4-7). (C) Effect of ATP on excitability of isolated optic nerves. Compound action potential (CAP) area remained stable after 180 min at 37°C whereas exposure to ATP (10 mM, n= 5) caused a 50% reduction in CAP area (CAP area was normalized to 100% at time 0). * p<0.05, * * p<0.01.

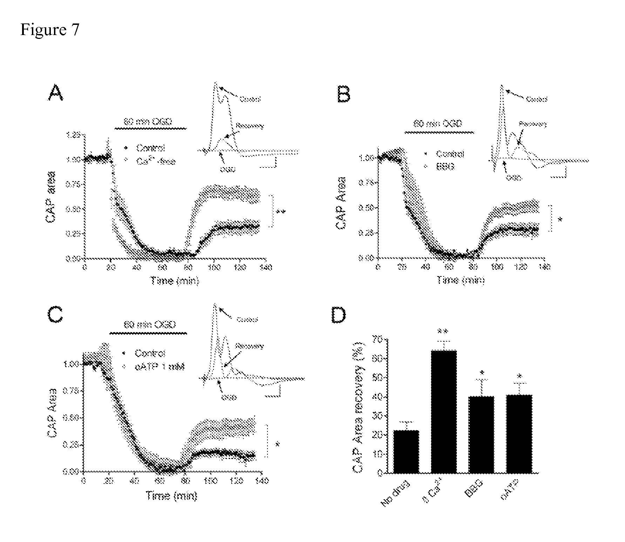

**Figure 7****.** P2X7 receptor antagonist prevents loss of white matter function after OGD. (A) Normalized CAP area recorded before, during and after 60 min OGD in drug-free aCSF (control; n= 4) and in aCSF with no Ca²⁺ (plus 200 µM EGTA and 4 mM Mg²⁺; n=4). **(B, C)** Normalized CAP area recorded before, during and after 60 min OGD in drug-free aCSF (control; n=15), in aCSF plus BBG (50 nM; n=7) and plus o-ATP (1mM; n=6). Inserts in *A, B,* C are representative traces of CAPs before, during and after the ischaemic insult. Scale bar = 1 mV, 1 ms. (D) Bar graph summarizing the extent of CAP recovery 60 min after 1 h OGD in control conditions (no drug), in the absence of extracellular Ca²⁺ and in the presence of P2X7R antagonists. *p<0.05, * *p<0.005.

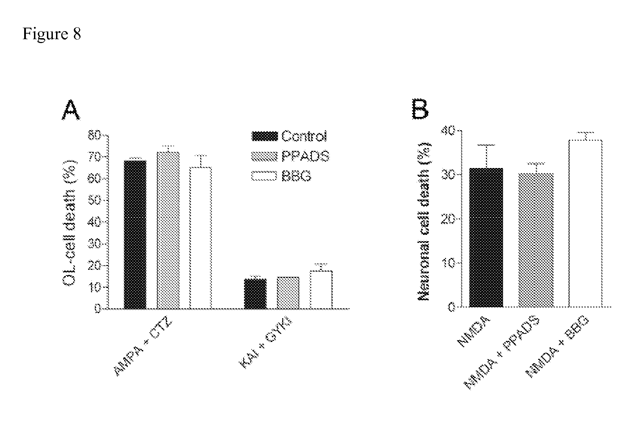

**Figure 8****.** P2X7 does not alter glutamate receptor-mediated cell death. (A) Oligodendrocyte cell death, as analyzed by calcein fluorescence (see methods), after 15 5 min exposure to glutamate receptor agonists AMPA (100 µM) in the presence of cyclothiazide (CTZ, 100 µM), and kainate (3 mM) plus GYKI 53655 (50 µM) to selectively activate AMPA and kainate receptors respectively. P2X7 antagonists PPADS (100 µM) and BBG (50 nM) were applied 5 min prior and during exposure to glutamate receptor agonists, and they did not significantly inhibit glutamate receptor-induced oligodendrocyte death. (B) Neuronal death, analyzed by calcein fluorescence (see methods), after 30 min exposure to NMDA (100 µM) plus glycine (10 µM) in culture medium lacking Mg 2+. P2X7 antagonists were applied as below and did not affect to NMDA-induced neuronal death. Neuronal cultures were obtained from cortical lobes of E18 embryos as previously described (Gottlieb et al., 2006. Neurobiol Dis 23:374-386.). Values are the mean ± SEM of at least three different experiments performed in triplicate.

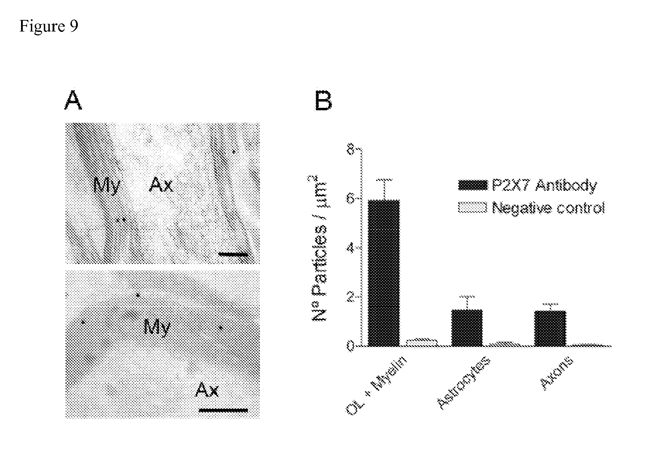

**Figure 9****.** Histogram showing the distribution of gold particles observed at the EM after staining of P2X7 receptors. (A) Representative images of longitudinal (top) and transversal (bottom) sections showing immunogold staining of P2X7 in the optic nerve. Ax= axon; My= myelin. Scale bar= 100 nm. (B) Histogram showing the distribution of P2X7 in the optic nerve. Quantification was performed by taking microphotographs (n=122) from three independent experiments. Scion Image software (National Institutes of Health, Frederick, MD) was used to measure the area of each cellular element and the number of gold particles inside them, and thus to calculate particle density (particles per square micrometer). Immunolabeling was absent when omitting the primary antibody. Experimental procedures for EM immunogold were as described earlier (Matute et al., 2007. J Neurosci 27:9525-9533).OL= oligodendrocyte.

### DETAILED DESCRIPTION OF THE INVENTION

The authors of the present invention have surprisingly found that following ischemia, oligodendrocytes and white matter tracts release ATP through pannexin hemichannels at levels sufficient to activate low affinity P2X7 receptors which kill oligodendrocytes, and that P2X7 antagonists and pannexin hemichannel blockers are protective in *both in vitro* and *in situ* models of white matter ischemia. This finding opens the possibility for improved therapies for ischemia by using pannexin blockers.

Thus, in a first aspect, the invention relates to a pannexine hemichannel inhibitor for use in the treatment of central nervous system ischemia, or, in other words, to the use of a pannexine hemichannel inhibitor for the preparation of a medicament for the treatment of central nervous system ischemia.

Although a variety of subjects can be treated, it is particularly preferred that the subject be a human being.

"Pannexin hemichannels" or "pannexins", as used herein, refers to proteins which form pores in the membranes and permits the release of ATP. Pannexins are members of the hemichannels or connexions familiy. So far, three pannexins have been described in human and rodent genomes: PANX1, PANX2 and PANX3. Hypothetical roles of pannexins include participating in sensory processing, hippocampal plasticity, synchronization between neurons, and propagation of the calcium waves supported by glial cells. Pannexins also may participate in pathological reactions in the central nervous system.

Pannexin-encoding genes are highly conserved in worms, mollusks, insects, and mammals. In humans, PANX1 (NCBI Reference Sequence NM_015368, SEQ ID NO: 1) and PANX3 (NCBI Reference Sequence NM_052959, SEQ ID NO: 2) genes are located on chromosome 11, at 30 Mb distance from each other, while PANX2 (NCBI Reference Sequence NM_052839.3, SEQ ID NO: 3) is located on chromosome 22 (Baranova A. et al. 2004. Genomics, 83(4):706-7162). All PANX1, PANX2 and PANX3 genes have been cloned (Baranova A. et al. 2004. Genomics, 83(4):706-7162). They have been predicted to have four transmembrane regions, two extracellular loops, one intracellular loop and intracellular C and N termini.

So far, PANX1 gene was found to form functional channel both alone and in combination with PANX 2, whereas PANX 2 alone was not able to form a channel (Bruzzone R. et al. 2003. Proc Natl Acad Sci USA.; 100:13644-13649). Homomeric and heteromeric hemichannels can differ in their gating properties.

Pannexin channels have been shown to be involved in ATP-induced ATP release, which is typical for calcium wave's propagation. PANX1 can form functional hemichannels at physiologic calcium concentrations and is expressed in many organs and tissues capable of calcium wave's propagations Bruzzone R. et al. 2003. Proc Natl Acad Sci USA.; 100:13644-13649). Calcium waves are supported by glial cells, which can maintain and modulate neuronal metabolism.

As "central nervous system ischemia" (CNS ischemia) in the present invention is understood as a restriction in blood supply, generally due to factors in the blood vessels, with resultant damage or dysfunction of a CNS tissue. The CNS comprises the brain and the spinal cord.

Rather than hypoxia (a more general term denoting a shortage of oxygen, usually a result of lack of oxygen in the air being breathed), ischemia is an absolute or relative shortage of the blood supply to an organ, i.e. a shortage of oxygen, glucose and other blood-borne fuels. A relative shortage means the mismatch of blood supply (oxygen/fuel delivery) and blood request for adequate metabolism of tissue. Ischemia results in tissue damage because of a lack of oxygen and nutrients. Ultimately, this can cause severe damage because of the potential for a build-up of metabolic wastes. "Ischemia" can also be described as an inadequate flow of blood to a part of the body, in this case the CNS, caused by constriction or blockage of the blood vessels supplying it. This inadequate blood flow or lack of oxygen or nutrients can be due, among others things, to
- Hypoglycemia (lower than normal level of blood glucose)
- Tachycardia (abnormally rapid beating of the heart)
- Atherosclerosis (lipid-laden plaques obstructing the lumen of arteries)
- Hypotension (low blood pressure, e.g. in septic shock, heart failure)
- Thromboembolism (blood clots)
- Outside compression of a blood vessel, e.g. by a tumor or in the case of Superior mesenteric artery syndrome
- Embolism (foreign bodies in the circulation, e.g. amniotic fluid embolism)
- Sickle cell disease (abnormally shaped red blood cells)
- Induced g-forces which restrict the blood flow and force the blood to the extremities of the body, as in acrobatics and military flying
- Localized extreme cold, such as by frostbite, ice, or improper Cold compression therapy
- Vscular dementia in aging.

Ischemia is a feature of heart diseases, transient ischemic attacks, cerebrovascular accidents, ruptured arteriovenous malformations, and peripheral artery occlusive disease. The heart, the kidneys, and the brain are among the organs that are the most sensitive to inadequate blood supply. Ischemia in brain tissue, for example due to stroke or head injury, causes a process called the ischemic cascade to be unleashed, in which proteolytic enzymes, reactive oxygen species, and other harmful chemicals damage and may ultimately kill brain tissue.

In a preferred embodiment, the ischemia is a "white matter ischemia". "White matter ischemia" in the present invention refers to an ischemia, as it was described previously, that occurs in the CNS (brain or spinal cord) zones denominated white matter. White matter is one of the two components of the CNS and consists mostly of myelinated axons which connect various grey matter areas (the locations of nerve cell bodies) of the brain or spinal cord. The major cellular components of white matter are glial cells: oligodendrocytes and astrocytes. Thus, white matter injury caused by white matter ischemia also involves an important contribution of brain glial cells (oligodendrocytes and astrocytes). During white matter ischemia, the decrease in O₂ and glucose triggers oligodendrocytes cell death.

Different methods can be used for distinguish between gray and white matter ischemia such as Diffusion-weighted imaging (DWI). DWI is a valuable MRI technique for evaluation of clinical stroke because it is highly sensitive to early ischemic changes (Neumann-Haefelin T, et al. Ann Neurol. 2000; 47:559-570). DWI can provide early identification of subcortical infarcts and white matter injury after global cerebral hypoxia or ischemia (Chalela JA, et al. Neurology. 2001; 56:481-485). DWI is sensitive to the thermal movement of water molecules. Although such motion is random, it may be directionally sensitive, or anisotropic, according to the microscopic organization of the surrounding tissue. Diffusion tensor imaging (DTI) is a new imaging technique that allows measurement of the magnitude and direction of signal anisotropy. Because many white matter regions are organized in parallel fiber arrays with high anisotropy, DTI can distinguish gray and white matter and may provide unique information regarding brain white matter structure and development. In combination with functional MRI, DTI can be used to track functional fiber connections in the human brain. Current studies indicate that DTI is highly sensitive to white matter stroke (Mukherjee P, et al. Radiology. 2000; 215:211-220; Sorensen AG, et al. Radiology. 1999; 212:785-792). Therefore, DTI may prove especially valuable for characterizing early white matter vulnerability to cerebral ischemia.

### Pannexin hemichannel inhibitors

"Pannexin hemichannel inhibitors", as used herein, relates to any compound capable of causing a decrease in the pannexin hemichannel activity, including those compounds which prevent expression of the pannexin hemichannel gene, leading to reduced pannexin hemichannel mRNA or protein levels as well as compounds that inhibit pannexin hemichannel causing a decrease in the ATP flow through the hemichannel. In a particular embodiment, the pannexine hemichannel inhibitor is a pannexin hemichannel blocker. A pannexin hemichannel blocker as used herein relates to any compound capable of binding to the pannexine hemichannel molecule, either inside of the pore or at other part of the hemichannel and block the flow of ATP through it.

Compounds leading to the inhibition of a pannexin hemichennel can be identified using standard assays such as methods for measuring the ATP flow through the hemichannel, like the method described by Bruzzone R. et al. (Bruzzone R. et al. 2005. Journal of Neurochemistry Volume 92 Issue 5, Pages 1033 - 1043), or using ATP biosensors (Anselmi F, et al. Proc Natl Acad Sci U S A. 2008 Dec 2;105(48):18770-5), as well as with the method disclosed herein (see Example, "Methods", "ATP release measurement"), wherein the ATP release from the cell is measured using a luciferin/luciferase assay kit.

The determination of the inhibiting or blocking capacities of the compound on the biological activity of pannexins is detected using standard assays to measure the ATP release using the methods cited above.

Exemplary, non-limitative, pannexin hemichannel inhibitors that can be used in the treatment of central nervous system ischemia according to the instant invention are described under I to XII in Table 1.

**Table 1**

| **Pannexin hemichannels inhibitors** | |
|---|---|
| | Compound |
| I | Carbenoxolone (CBX) [2S,4aS,6aR,6aS,6bR,8aR,10S,12aS, 14bR)-10-(4-hydroxy-4-oxobutanoyl)oxy-2,4a,6a,6b,9,9,12a-heptamethyl-13-oxo-3,4,5,6,6a, 7,8,8a,10,11,12,14b-dodeca-hydro-1H-picene-2-carboxylic acid) or a pharmaceutically acceptable salt thereof, e.g., the disodium salt of formula |
| | 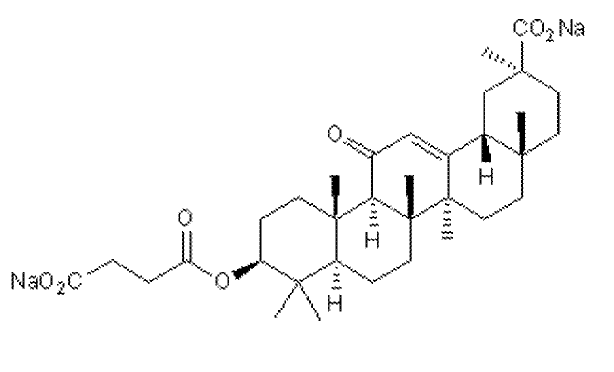 |
| | Carbenoxolone disodium [IC₅₀ about 5 µM] [Bruzzone R. et al. 2005. Journal of Neurochemistry 92(5):1033-1043] |
| II | A compound of general formula: |
| | 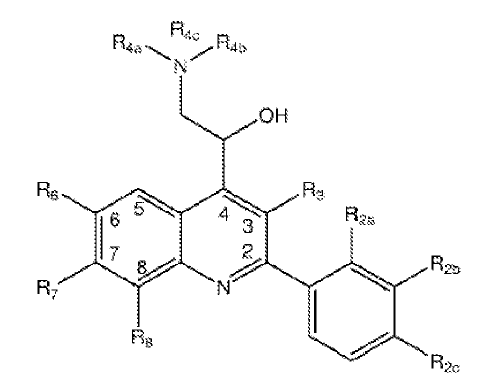 |
| | wherein |
| | R₂ₐ represents Cl, H, OH, a trifluoromethyl group, or a t-butyl group; |
| | R_{2b} represents Cl, H, or OH; |
| | R_{2c} represents Cl, F, H, I or OH; |
| | R₃ represents H, F or Cl; |
| | R₄ₐ represents butyl or hexyl; |
| | R_{4b} represents H, ethyl, butyl or hexyl; |
| | R₆ represents H, Cl, CF₃ or methyl; |
| | R₇ represents H or Cl; and |
| | R₈ represents H, Cl or methyl. |
| | [Dow G. S. et al 2006. Antimicrobial agents and chemotherapy. 50:4132-4143] |
| III | A compound of general formula: |
| | 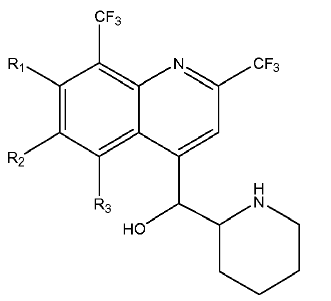 |
| | wherein |
| | R₁ represents H, Cl, CF₃ or methyl; |
| | R₂ represents H or Cl; and |
| | R₃ represents H, Cl or methyl. |
| IV | 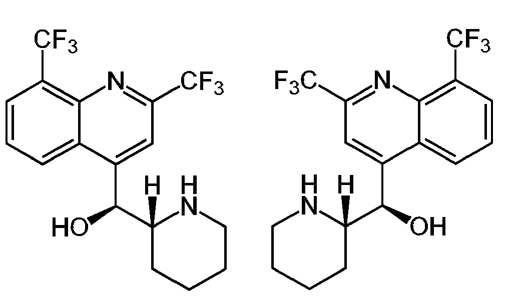 |
| | Mefloquine (MFQ) [8-bis(trifluoromethyl)quinolin-4-yl]-[(2R)-piperidin-2-yl]methanol or a pharmaceutically acceptable salt thereof (e.g., hydrochloride) |
| V | 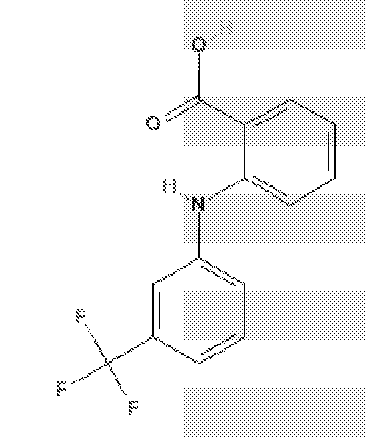 |
| | Flufenamic acid (FFA) ]2-[3-(trifluoromethyl)anilino]benzoic acid] |
| VI | 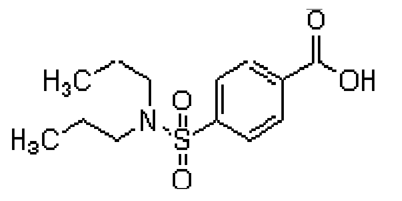 |
| | Probenecid [p-(dipropylsulfamoyl)benzoic acid] |
| | [Silverman W., et al. Am J Physiol Cell Physiol. 2008 September; 295(3): C761-C767] |
| VII | An oleamide of formula |
| | 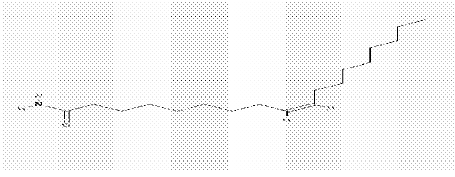 (Z)-octadec-9-enamide |
| | 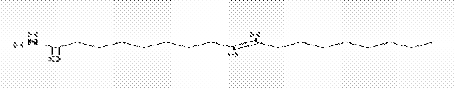 (E)-octadec-9-enamide |
| | [Buvinic et al 2009 JBC Published on October 12, 2009 as Manuscript M109.057315] |
| VIII | An inhibitory antibody capable of specifically binding and inhibiting the activity of a pannexin hemichannel |
| IX | A ribozyme or a DNA enzyme specific for the sequence of a pannexin hemichannel |
| X | An interfering RNA specific for the sequence of a pannexin hemichannel |
| XI | An inhibiting peptide specific for a pannexin hemichannel |
| XII | A pannexin hemichannel-specific antisense nucleic acid |

### Pannexin Inhibitory antibodies (VIII)

Antibodies against an epitope located in either the pore or an external part of the pannexin molecule may effectively inhibit the function of this protein and, therefore, can be used as blocker or inhibitor in the compositions of the present invention. "inhibitory antibody", as used herein, refers to antibodies which are capable of inhibiting at least partially the biological activities of the pannexin hemichannels.

The determination of the inhibiting capacity on the biological activity of the pannexin hemichannels is detected using standard assays to measure the ATP release through the pannexin hemichannels using method described before.

Inhibiting antibodies or fragments specific for pannexin hemichannels may be readily available, or may be readily produced using conventional molecular biology techniques. For example, using immunogens derived from, for example, pannexin hemichannel it is possible to obtain anti-protein/anti-peptide antisera or monoclonal antibodies by using standard protocols (See, for example, Antibodies: A Laboratory Manual ed. by Harlow and Lane (Cold Spring Harbor Press: 1988)). A mammal, such as a mouse, a hamster or rabbit can be immunized with an immunogenic form of the peptide (e.g., pannexin hemichannel or an antigenic fragment thereof, which is capable of eliciting an antibody response). Techniques for conferring immunogenicity on a protein or peptide, include conjugation to carriers or other techniques, are well known in the art. An immunogenic portion of a polypeptide can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the antibodies forming part of the compositions of the invention are immuno-specific for antigenic determinants of pannexin hemichannel (or a variant at least 80%, 85%, 90%, 95%, or 98% identical thereto). In certain embodiment, the immunospecific subject antibodies do not substantially cross react with a non-vertebrate (such as yeast) pannexin hemichannel related protein. By "not substantially cross react", it is meant that the antibody has a binding affinity for a non-homologous protein which is at least one order of magnitude, more preferably at least 2 orders of magnitude, and even more preferably at least 3 orders of magnitude less than the binding affinity of the antibody for a pannexin hemichannel.

Thus, the antibody which can be used for the purposes of the instant invention as a pannexin inhibitory antibody, hereinafter referred to as the "antibody of the invention", is capable of binding an epitope of the pannexin hemichannel; typically, at least 6, 8, 10, or 12, contiguous amino acids are required to form an epitope, however, epitopes which involve non-contiguous amino acids may require more, e.g., at least 15, 25, or 50 amino acid. The term "antibody of the invention" includes, for example, polyclonal antibodies, monoclonal antibodies, Fab and single chain Fv (scFv) fragments thereof, bispecific antibodies, heteroconjugates, human and humanized antibodies. Such antibodies may be produced in a variety of ways, including hybridoma cultures, recombinant expression in bacteria or mammalian cell cultures, and recombinant expression in transgenic animals. Also antibodies can be produced by selecting a sequence from a library of sequences expressed in display systems such as filamentous phage, bacterial, yeast or ribosome. There is abundant guidance in the literature for selecting a particular production methodology, e.g., Chadd and Chamow, Curr. Opin. Biotechnol., 12:188-194 (2001). The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, having no effector functions and limited pharmokinetic activity may be generated in a bacterial expression system. Single chain Fv fragments show low immunogenicity and are cleared rapidly from the blood.

The antibodies of the invention may be polyclonal antibodies. Such polyclonal antibodies can be produced in a mammal, such as a non-human mammal, for example, following one or more injections of an immunizing agent, and preferably, an adjuvant. Typically, the immunizing agent and/or adjuvant will be injected into the mammal by a series of subcutaneous or intraperitoneal injections. The immunizing agent may include pannexin hemichannels or fragments thereof or a fusion protein thereof or a cell expressing pannexin hemichannels. Such proteins, fragments or preparations are introduced into the non-human mammal in the presence of an appropriate adjuvant. Other form of administration of an immunogen is as a trasmembrane protein in the surface of a cell (methods described in, e.g., Spiller et al. J. Immunol. Methods, 224: 51-60 (1999)). These cells can be either cells which naturally express the antigen or in which this expression can be obtained after transfecting the cell with a DNA construct that contains among other DNA sequences those coding the antigen, those necessary for its sufficient expression in the cell. This approach is possible not only when the cell membrane is the natural site in which the antigen is expressed even the antigen once synthesized in the cell is directed at these location by a signal peptide which is added at the antigen coding sequence. If the serum contains polyclonal antibodies to undesired epitopes, the polyclonal antibodies can be purified by immunoaffinity chromatography.

Alternatively, said antibodies may be monoclonal antibodies. Monoclonal antibodies may be produced by hybridomas, wherein a mouse, hamster, or other appropriate host animal, is immunized with an immunizing agent to elicit lymphocytes that produce or are capable of producing antibodies that will specifically bind to the immunizing agent, e.g. Kohler and Milstein, Nature 256:495 (1975). The immunizing agent will typically include a pannexin hemichannel or a fragment thereof or a fusion protein thereof and optionally a carrier or a crude protein preparation which has been enriched for a pannexin hemichannel or a cell expressing any of said proteins. Such proteins, fragments or preparations are introduced into the non-human mammal in the presence of an appropriate adjuvant. Other form of administration of an immunogen is as a trasmembrane protein in the surface of a cell (methods described in, e.g., Spiller et al. J. Immunol. Methods, 224: 51-60 (1999)). These cells can be everyone which naturally express the antigen in its cell membrane or in which this expression can be obtained after transfecting the cell with a DNA construct that contains among other DNA sequences those coding the antigen, those necessary for its sufficient expression in the cell. This approach is possible not only when the cell membrane is the natural site in which the antigen is expressed even the antigen once synthesized in the cell is directed at these location by a signal peptide which is added at the antigen coding sequence. Alternatively, lymphocytes may be immunized in vitro. Generally, spleen cells or lymph node cells are used if non-human mammalian sources are desired, or peripheral blood lymphocytes ("PBLs") are used if cells of human origin are desired. The lymphocytes are fused with an immortalized cell line using a suitable fusing agent, such as polyethylene glycol, to produce a hybridoma cell. In general, immortalized cell lines are myeloma cells of rat, mouse, bovine or human origin. The hybridoma cells are cultured in a suitable culture medium that preferably contains one or more substances that inhibit the growth or survival of unfused, immortalized cells. Clones are isolated using the limiting dilution method and the culture medium (supernatant) in which the hybridoma cells are cultured can be assayed for the presence of monoclonal antibodies directed against pannexin hemichannels by conventional techniques, such as by flow cytometry or by immunoprecipitation or by other *in vitro* binding assay, such as RIA or ELISA. Clones can also be cultured *in vivo* as ascites tumours in an animal.

Preferably, the binding specificity of monoclonal antibodies produced by a clone of hybridoma cells is determined by immunoprecipitation or by an in vitro binding assay, such as radioimmunoassay (RIA) or enzyme-linked immunoabsorbent assay (ELISA) or by immunofluorescent techniques such as fluorescence microscopy or flow cytometry. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in US 4,816,567. DNA encoding the monoclonal antibodies of the invention can be isolated from a pannexin hemichannel-specific hybridoma cells and sequenced by using conventional procedures, e.g., by using oligonucleotide probes that are capable of binding specifically to genes encoding the heavy and light chains of murine antibodies. The hybridoma cells serve as a preferred source of such DNA. Once isolated, the DNA may be inserted into an expression vector, which is then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for the murine heavy and light chain constant domains for the homologous human sequences, or by covalently joining to the immunoglobulin coding sequence all or part of the coding sequence for a non-immunoglobulin polypeptide. The non-immunoglobulin polypeptide can be substituted for the constant domains of an antibody of the invention, or can be substituted for the variable domains of one antigen-combining site of an antibody of the invention to create a chimeric bivalent antibody.

Another method of generating specific antibodies, or antibody fragments, reactive against a target molecule is to screen expression libraries encoding immunoglobulin genes, or portions thereof, expressed in bacteria, yeast, filamentous phages, ribosomes or ribosomal subunits and other display systems. These methods normally use large libraries of antibody sequences or antibody fragment sequences obtained from diverse sources such healthy donors or patients or animals healthy or not. These sequences are cloned and expressed in an appropriate system and selected by its binding affinity for the antigen. Diverse approaches have been described to select antibodies or fragments with desired properties e.g. neutralizing, agonist, etc (Fernández, Curr. Op. Biotech., 15: 364-373 (2004); Schmidt, Eur. J. Biochem., 268: 1730-1738 (2001)). In an embodiment, antibodies and antibody fragments characteristic of hybridomas of the invention can also be produced by recombinant means by extracting messenger RNA, constructing a cDNA library, and selecting clones which encode segments of the antibody molecule.

The antibodies may also be engineered to alter its clinical uses. Numerous approaches make use of the molecular biology and genetic techniques such as the good knowledge of the genetics ad structure of the immunoglobulins to construct different modifications of immunoglobulin molecule with the aim of improve its properties for clinical or other uses. Some of them tend to reduce the immunogenicity of the molecule in the species in which should be used and the resultant molecule has a sequence more homologous with this species. Various methods have been used to obtain mAbs of human origin avoiding the non ethically admissible proceedings in healthy humans. In other approaches the molecular weigh an size are reduced e.g. in order of improving the distribution of the molecule into solid tumours. Other possibilities are conjugation in a molecule of binding domains for more than one target molecule (bispecific antibody or also triespecific, etc) or the conjugation of an antibody or a fragment with another molecule with the desired function e.g. a toxic agent, a hormone, growth factor, a immunomodulating agent (immunosuppressor or immunostimulator), an inhibitor of cell growth, etc. In general all the resultant molecules retain almost one variable domain of an antibody which gives the high specificity and affinity characteristic of the antigen-antibody binding. Some examples of these constructions are:
- Chimeric antibodies: These refers to antibodies constructed with variable regions from an antibody of some species (normally a mammal in which the mAb was generated) and constant regions of other species (the one in which the chimeric antibody is to be used);
- Humanized antibodies: By "humanized antibody" is meant an antibody derived from a non-human antibody, typically a murine antibody, that retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues. Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Reichmann et al., Nature, 332:323-327 (1988); Verhoeyen et al., Science, 239:1534-1536 (1988)), by substituting hypervariable region sequences for the corresponding sequences of a human antibody. (Carter et al., Proc. Natl. Acad. Sci. USA, 89:4285 (1992); Presta et al., J. Immunol., 151:2623 (1993)). It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences.
- Primatized antibodies: A next step in this approach of making an antibody more similar at humans' are the so called primatized antibodies, i.e. a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). The preparation of such antibodies is described in Newman et al., Biotechnology, 10: 1458-1460 (1992); US 5,658,570 and US 6,113,898. These antibodies have been reported to exhibit a high degree of homology to human antibodies, i.e., 85-98%, display human effector functions, have reduced immunogenicity, and may exhibit high affinity to human antigens. Another highly efficient means for generating recombinant antibodies is disclosed by Newman, Biotechnology, 10: 1455-1460 (1992).
- Human antibodies: By "human antibody" is meant an antibody containing entirely human light and heavy chain as well as constant regions, produced by any of the known standard methods. As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH) gene in chimeric and germ-line mutant mice results in complete inhibition of endogenous antibody production. Transfer of the human germ-line immunoglobulin gene array in such germ line mutant mice will result in the production of human antibodies after immunization. See, e.g., Jakobovits et al., Proc. Mad. Acad. Sci. USA, 90:255 1 (1993); Jakobovits et al., Nature, 362:255-258 (1993). Alternatively, phage display technology (McCafferty et al., Nature 348:552-553 (1990)) can be used to produce human antibodies and antibody fragments in vitro, from immunoglobulin variable (V) domain gene repertoires from donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson, Kevin S. and Chiswell, David J., Current Opinion in Structural Biology 3:564-57 1 (1993). Human antibodies may also be generated by in vitro activated B cells or SCID mice with its immune system reconstituted with human cells. Once obtained a human antibody, its coding DNA sequences can be isolated, cloned and introduced in an appropriate expression system i.e. a cell line preferably from a mammal which subsequently express and liberate into culture media from which the antibody can be isolated.
- Antibody fragments: An antibody fragment is a fragment of an antibody such as, for example, Fab, F(ab')2, Fab' and scFv. Various techniques have been developed for the production of antibody fragments. Traditionally, these fragments were derived via proteolytic digestion of intact antibodies but more recently these fragments can be produced directly by recombinant host cells. In other embodiments, the antibody of choice is a single chain Fv (scFv) fragment which additionally may be monospecific or bispecific. Papain digestion of antibodies produces two identical antigen-binding fragments, called "Fab" fragments, each with a single antigen-binding site, and a residual "Fc" fragment, which name reflects its ability to crystallize readily. Pepsin treatment yields an F(ab')2 fragment that has two antigen-binding sites and is still capable of cross-linking antigen. "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site. The Fab fragment also contains the constant domain of the light chain and the first constant domain (CHI) of the heavy chain. Fab' fragments differ from Fab fragments by the addition of a few residues at the carboxy terminus of the heavy chain CHI domain including one or more cysteines from the antibody hinge region. Fab'-SH is the designation herein for Fab' in which the cysteine residue(s) of the constant domains bear at least one free thiol group. F(ab')Z antibody fragments originally were produced as pairs of Fab' fragments which have hinge cysteines between them. Other chemical couplings of antibody fragments are also known. "Single-chain Fv" or "scFv" antibody fragments comprise the VH and VL domains of an antibody, wherein these domains are present in a single polypeptide chain. Preferably, the Fv polypeptide further comprises a polypeptide linker between the VH and VL domains which enables the scFv to form the desired structure for antigen binding. For a review of scFv see Pluckthun in The Pharmacology of Monoclonal Antibodies, vol. 113, Rosenburg and Moore eds., Springer-Verlag, N.Y., pp. 269-315 (1994). The term "diabodies" refers to small antibody fragments with two antigen-binding sites, which fragments comprise a heavy-chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).
- Bispecific antibodies: Bispecific antibodies are antibodies that have binding specificities for at least two different epitopes. Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J, 10:3655-3659 (1991).

### Pannexin hemichannel-specific ribozymes (IX)

Ribozyme molecules designed to catalytically cleave a target mRNA transcripts can also be used to prevent translation of pannexin hemichannel mRNAs. Accordingly, in another embodiment, the compositions of the invention comprise ribozymes specifically directed to the pannexin hemichannel mRNA. Ribozymes are enzymatic RNA molecules capable of catalyzing the specific cleavage of RNA. (For a review, see Rossi, Current Biology 4:469-471, 1994). The mechanism of ribozyme action involves sequence specific hybridization of the ribozyme molecule to complementary target RNA, followed by an endonucleolytic cleavage event. The composition of ribozyme molecules preferably includes one or more sequences complementary to a target mRNA, and the well known catalytic sequence responsible for mRNA cleavage or a functionally equivalent sequence (see, e.g., U.S. Pat. No. 5,093,246, incorporated herein by reference in its entirety).

While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. Preferably, the target mRNA has the following sequence of two bases: 5'-UG-3'. The construction and production of hammerhead ribozymes is well known in the art and is described more fully in Haseloff and Gerlach, Nature 334: 585-591, 1988; and see PCT Appln. No. W089/05852, the contents of which are incorporated herein by reference. Hammerhead ribozyme sequences can be embedded in a stable RNA such as a transfer RNA (tRNA) to increase cleavage efficiency in vivo (Perriman et al., Proc. Natl. Acad. Sci. USA, 92: 6175-79, 1995; de Feyter, and Gaudron, Methods in Molecular Biology, Vol. 74, Chapter 43, "Expressing Ribozymes in Plants," Edited by Turner, P. C, Humana Press Inc., Totowa, N.J.). In particular, RNA polymerase 111-mediated expression of tRNA fusion ribozymes are well known in the art (see, Kawasaki et al., Nature 393: 284-9, 1998; Kuwabara et al., Nature Biotechnol. 16: 961-5, 1998; and Kuwabara et al., Mol. Cell. 2: 617-27, 1998; Koseki et al., J Virol 73: 1868-77, 1999; Kuwabara et al., Proc Natl Acad Sci USA 96: 1886-91, 1999; Tanabe et al., Nature 406: 473-4, 2000). There are typically a number of potential hammerhead ribozyme cleavage sites within a given target CDNA sequence. Preferably the ribozyme is engineered so that the cleavage recognition site is located near the 5' end of the target mRNA to increase efficiency and minimize the intracellular accumulation of non-functional mRNA transcripts. Furthermore, the use of any cleavage recognition site located in the target sequence encoding different portions of the C-terminal amino acid domains of, for example, long and short forms of target would allow the selective targeting of one or the other form of the target, and thus, have a selective effect on one form of the target gene product.

Gene targeting ribozymes necessarily contain a hybridizing region complementary to two regions, each of at least 5 and preferably each 6, 7, 8, 9, 10, 11, 12, 13, 14, 15, 16, 17, 18, 19 or 20 contiguous nucleotides in length of a target mRNA, such as an mRNA of a sequence represented in the pannexin hemichannel genes. In addition, ribozymes possess highly specific endoribonuclease activity, which autocatalytically cleaves the target sense mRNA. The present invention extends to ribozymes which hybridize to a sense mRNA encoding a target gene such as a therapeutic drug target candidate gene, thereby hybridizing to the sense mRNA and cleaving it, such that it is no longer capable of being translated to synthesize a functional polypeptide product.

The ribozymes used in the compositions of the present invention also include RNA endoribonucleases (hereinafter "Cech-type ribozymes") such as the one which occurs naturally in Tetrahymena thermophila (known as the IVS, or L-19 IVS RNA) and which has been extensively described by Thomas Cech and collaborators (Zaug et al., Science 224:574-578, 1984; Zaug et al., Science 231: 470-475, 1986; Zaug et al., Nature 324: 429-433, 1986; published International patent application No. WO88/04300 by University Patents Inc.; Been, et al., Cell 47: 207-216, 1986). The Cech-type ribozymes have an eight base pair active site which hybridizes to a target RNA sequence whereafter cleavage of the target RNA takes place. The invention encompasses those Cech-type ribozymes which target eight base-pair active site sequences that are present in a target gene or nucleic acid sequence.

Ribozymes can be composed of modified oligonucleotides (e.g., for improved stability, targeting, etc.) and should be delivered to cells which express the target gene in vivo. A preferred method of delivery involves using a DNA construct "encoding" the ribozyme under the control of a strong constitutive pol III or pol II promoter, so that transfected cells will produce sufficient quantities of the ribozyme to destroy endogenous target messages and inhibit translation. Because ribozymes, unlike antisense molecules, are catalytic, a lower intracellular concentration is required for efficiency.
In certain embodiments, a ribozyme may be designed by first identifying a sequence portion sufficient to cause effective knockdown by RNAi. The same sequence portion may then be incorporated into a ribozyme. In this aspect of the invention, the gene-targeting portions of the ribozyme or RNAi are substantially the same sequence of at least 5 and preferably 6, 7, 8, 9, 10, 11, 12, 13, 14, 15, 16, 17, 18, 19 or 20 or more contiguous nucleotides of a target nucleic acid, such as a nucleic acid of any of the human acid ceramidase or choline kinase sequences. In a long target RNA chain, significant numbers of target sites are not accessible to the ribozyme because they are hidden within secondary or tertiary structures (Birikh et al., Eur J Biochem 245: 1-16, 1997). To overcome the problem of target RNA accessibility, computer generated predictions of secondary structure are typically used to identify targets that are most likely to be single-stranded or have an "open" configuration (see Jaeger et al., Methods Enzymol 183: 281-306, 1989). Other approaches utilize a systematic approach to predicting secondary structure which involves assessing a huge number of candidate hybridizing oligonucleotides molecules (see Milner et al., Nat Biotechnol 15: 537-41, 1997; and Patzel and Sczakiel, Nat Biotechnol 16: 64-8, 1998). Additionally, U.S. Pat. No. 6,251,588, the contents of which are hereby incorporated herein, describes methods for evaluating oligonucleotide probe sequences so as to predict the potential for hybridization to a target nucleic acid sequence. The method of the invention provides for the use of such methods to select preferred segments of a target mRNA sequence that are predicted to be single-stranded and, further, for the opportunistic utilization of the same or substantially identical target mRNA sequence, preferably comprising about 10-20 consecutive nucleotides of the target mRNA, in the design of both the RNAi oligonucleotides and ribozymes of the invention.

### Pannexin hemichannel-specific RNA interference (RNAi) (X)

In another embodiment, the inhibitors of the pannexin hemichannel that form part of the compositions of the invention are RNAi which are capable of knocking down the expression of a pannexin hemichannel or any component gene necessary for a pannexin hemichannel function. RNAi is a process of sequence-specific post-transcriptional gene repression which can occur in eukaryotic cells. In general, this process involves degradation of an mRNA of a particular sequence induced by double-stranded RNA (dsRNA) that is homologous to that sequence. For example, the expression of a long dsRNA corresponding to the sequence of a particular single-stranded mRNA (ss mRNA) will labilize that message, thereby "interfering" with expression of the corresponding gene. Accordingly, any selected gene may be repressed by introducing a dsRNA which corresponds to all or a substantial part of the mRNA for that gene. It appears that when a long dsRNA is expressed, it is initially processed by a ribonuclease III into shorter dsRNA oligonucleotides of as few as 21 to 22 base pairs in length. Accordingly, RNAi may be effected by introduction or expression of relatively short homologous dsRNAs. Indeed, the use of relatively short homologous dsRNAs may have certain advantages as discussed below.

The double stranded oligonucleotides used to effect RNAi are preferably less than 30 base pairs in length and, more preferably, comprise about 25, 24, 23, 22, 21, 20, 19, 18 or 17 base pairs of ribonucleic acid. Optionally the dsRNA oligonucleotides of the invention may include 3' overhang ends. Exemplary 2-nucleotide 3' overhangs may be composed of ribonucleotide residues of any type and may even be composed of 2'-deoxythymidine residues, which lowers the cost of RNA synthesis and may enhance nuclease resistance of siRNAs in the cell culture medium and within transfected cells (see Elbashir et al., Nature 411: 494-8, 2001). Longer dsRNAs of 50, 75, 100 or even 500 base pairs or more may also be utilized in certain embodiments of the invention. Exemplary concentrations of dsRNAs for effecting RNAi are about 0.05 nM, 0.1 nM, 0.5 nM, 1.0 nM, 1.5 nM, 25 nM or 100 nM, although other concentrations may be utilized depending upon the nature of the cells treated, the gene target and other factors readily discernable to the skilled artisan. Exemplary dsRNAs may be synthesized chemically or produced in vitro or in vivo using appropriate expression vectors. Exemplary synthetic RNAs include 21 nucleotide RNAs chemically synthesized using methods known in the art (e.g., Expedite RNA phosphoramidites and thymidine phosphoramidite (Proligo, Germany). Synthetic oligonucleotides are preferably deprotected and gel-purified using methods known in the art (see, e.g., Elbashir et al., Genes Dev. 15: 188-200, 2001). Longer RNAs may be transcribed from promoters, such as T7 RNA polymerase promoters, known in the art. A single RNA target, placed in both possible orientations downstream of an in vitro promoter, will transcribe both strands of the target to create a dsRNA oligonucleotide of the desired target sequence. Any of the above RNA species will be designed to include a portion of nucleic acid sequence represented in a target nucleic acid, such as, for example, a nucleic acid that hybridizes, under stringent and/or physiological conditions, to the polynucleotide encoding human a pannexin hemichannel.

The specific sequence utilized in design of the oligonucleotides may be any contiguous sequence of nucleotides contained within the expressed gene message of the target. Programs and algorithms, known in the art, may be used to select appropriate target sequences. In addition, optimal sequences may be selected utilizing programs designed to predict the secondary structure of a specified single stranded nucleic acid sequence and allowing selection of those sequences likely to occur in exposed single stranded regions of a folded mRNA. Methods and compositions for designing appropriate oligonucleotides may be found, for example, in U.S. Pat. No. 6,251,588, the contents of which are incorporated herein by reference. Messenger RNA (mRNA) is generally thought of as a linear molecule which contains the information for directing protein synthesis within the sequence of ribonucleotides, however studies have revealed a number of secondary and tertiary structures that exist in most mRNAs. Secondary structure elements in RNA are formed largely by Watson-Crick type interactions between different regions of the same RNA molecule. Important secondary structural elements include intramolecular double stranded regions, hairpin loops, bulges in duplex RNA and internal loops. Tertiary structural elements are formed when secondary structural elements come in contact with each other or with single stranded regions to produce a more complex three dimensional structure. A number of researchers have measured the binding energies of a large number of RNA duplex structures and have derived a set of rules which can be used to predict the secondary structure of RNA (see, e.g., Jaeger et al., Proc. Natl. Acad. Sci. USA 86: 7706, 1989; and Turner et al., Annu. Rev. Biophys. Biophys. Chem. 17:167, 1988). The rules are useful in identification of RNA structural elements and, in particular, for identifying single stranded RNA regions which may represent preferred segments of the mRNA to target for silencing RNAi, ribozyme or antisense technologies. Accordingly, preferred segments of the mRNA target can be identified for design of the RNAi mediating dsRNA oligonucleotides as well as for design of appropriate ribozyme and hammerhead ribozyme compositions of the invention.

Several different types of molecules have been used effectively in the RNAi technology. Small interfering RNA (siRNA), sometimes known as short interfering RNA or silencing RNA, are a class of 20-25 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g., as an antiviral mechanism or in shaping the chromatin structure of a genome. Synthetic siRNAs have been shown to be able to induce RNAi in mammalian cells. This discovery led to a surge in the use of siRNA/RNAi for biomedical research and drug development.

MicroRNA (miRNA) are a related class of gene regulatory small RNA_{S}, typically 21-23 nt in length. They typically differ from siRNA because they are processed from single stranded RNA precursors and show only partially complementary to mRNA targets. Initial studies have indicated that miRNAs regulate gene expression post-transcriptionally at the level of translational inhibition at P-Bodies in the cytoplasm. However, miRNAs may also guide mRNA cleavage similar to siRNAs. This is often the case in plants where the target sites are typically highly complementary to the miRNA. While target sites in plant mRNAs can be found in the 5'UTR, open-reading frames and 3'UTR, in animals, it is the 3' UTR that is the main target. miRNAs are first transcribed as part of a primary microRNA (pri-miRNA). This is then processed by the Drosha with the help of Pasha/DGCR8 (=Microprocessor complex) into pre-miRNAs. The ca. 75 nt pre-miRNA is then exported to the cytoplasm by exportin-5, where it is then diced into 21-23 nt siRNA-like molecules by Dicer. In some cases, multiple miRNAs can be found on the pri-miRNA.

Short hairpin RNA (shRNA) is yet another type of RNA that may be used to effect RNAi. It is a sequence of RNA that makes a tight hairpin turn that can be used to silence gene expression. shRNA is transcribed by RNA polymerase III.

Currently, short-interfering RNAs (siRNAs) and short-hairpin RNAs (shRNAs) are being extensively used to silence various genes to silence functions carried out by the genes. It is becoming easier to harness RNAi to silence specific genes, owing to the development of libraries of readymade shRNA and siRNA gene-silencing constructs by using a variety of sources. For example, RNAi Codex, which consists of a database of shRNA related information and an associated website, has been developed as a portal for publicly available shRNA resources and is accessible at http://codex.cshl.org. RNAi Codex currently holds data from the Hannon-Elledge shRNA library and allows the use of biologist-friendly gene names to access information on shRNA constructs that can silence the gene of interest. It is designed to hold user-contributed annotations and publications for each construct, as and when such data become available. Olson et al. (Nucleic Acids Res. 34(Database issue): D153-D157, 2006, incorporated by reference) have provided detailed descriptions about features of RNAi Codex, and have explained the use of the tool. All these information may be used to help design the various siRNA or shRNA targeting pannexin hemichannel or other proteins of interest.

Illustrative, non-limitative, examples of interfering RNA specific for the sequence of a pannexin hemichannel include those described by Locovei S, et al. (Locovei S et al., 2007 FEBS Lett. 581:483- 488), (Target sequences for the human pannexin1 siRNA: GGCUGCCUUUGUGGAUUCA (SEQ ID NO: 4) and GGAGAUCUCGAUUGGUACA (SEQ ID NO: 5). Target sequences for mouse pannexin1 siRNA: GGCUGCCUUUGUGGAUUCA (SEQ ID NO: 6) , GGAACUUGACAAAGUCUAC (SEQ ID NO: 7) and GGUGCUGGAGAACAUUAAA (SEQ ID NO: 8).

### Pannexin hemichannel specific-inhibiting peptides (XI)

A "pannexin hemichannel specific-inhibiting peptide" as used herein, relates to a peptide that biologically mimics regions of the protein sequence of the pannexin hemichannel that are involve in the pannexin activity and thus they would inhibit the physiological activity of the hemichannel.

The screening or design of a pannexin hemichannel specific-inhibiting peptide can be done as described in Olson, G.L et al (J. Med. Chem., 36, 3039-3049, 1993) or in Gillespie, P.et al. (Biopolymers, 43:191-217 1997).

Illustrative, non-limitative, examples of inhibiting peptides specific for a pannexin hemichannel include those described by Wang et al. (Wang et al., 2007 Am J Cell Physiol 293:1112-1119), which are hereby incorporated by reference, namely:
Panx1 : WRQAAFVDSY (SEQ ID NO: 9)
E1a: AQEISIGTQIS (SEQ ID NO: 10)
E1b: SSFSWRQAAFVDS (SEQ ID NO: 11)
E1c: SESGNLPLWLHK (SEQ ID NO: 12)
E2a: SSLSDEFVCSIKS (SEQ ID NO: 13)
E2b: KSGILRNDSTVPDQ (SEQ ID NO: 14)

A person skilled in the art would understand that the pannexin hemichannel can be formed by homomultimeric assemblies by using the same subunits (e.g. only Panx1, Panx2 or Panx3 subunits in one hemichannel), or in the form of heteromultimeric assemblies (e.g. with both Panx1 and Pax2 subunits in one hemichannel). Thus the inhibition of the pannexin hemichannel by the pannexin channel inhibitor is going to depend on the multimeric assembly of the hemichannel.

Thus, in an embodiment the pannexin hemichannel is selected from a homomultimeric hemichannel form by Panx1, Panx2 or Panx3 subunits. In another embodiment, the pannexin hemichannel is selected from heteromultimeric assemblies of Panx 1, Panx2 and Panx 3. In a preferred embodiment, the hemichannels is a homomultimeric hemichannel formed by Panx1 subunits.

### Pannexin hemichannel-specific antisense nucleic acids (XII)

In a further embodiment, the invention relates to the use of the isolated "antisense" nucleic acids to inhibit expression, e.g., by inhibiting transcription and/or translation of pannexin hemichannels nucleic acids. The antisense nucleic acids may bind to the potential drug target by conventional base pair complementarity, or, for example, in the case of binding to DNA duplexes, through specific interactions in the major groove of the double helix. In general, these methods refer to the range of techniques generally employed in the art, and include any methods that rely on specific binding to oligonucleotide sequences.

An antisense construct of the present invention can be delivered, for example, as an expression plasmid which, when transcribed in the cell, produces RNA which is complementary to at least a unique portion of the cellular mRNA which encodes a pannexin hemichannel polypeptide. Alternatively, the antisense construct is an oligonucleotide probe, which is generated ex vivo and which, when introduced into the cell causes inhibition of expression by hybridizing with the mRNA and/or genomic sequences of a target nucleic acid. Such oligonucleotide probes are preferably modified oligonucleotides, which are resistant to endogenous nucleases, e.g., exonucleases and/or endonucleases, and are therefore stable in vivo. Exemplary nucleic acid molecules for use as antisense oligonucleotides are phosphoramidate, phosphothioate and methylphosphonate analogs of DNA (see also U.S. Pat. Nos. 5,176,996; 5,264,564; and 5,256,775). Additionally, general approaches to constructing oligomers useful in antisense therapy have been reviewed, for example, by Van der Krol et al., BioTechniques 6: 958-976, 1988; and Stein et al., Cancer Res 48: 2659-2668, 1988.

With respect to antisense DNA, oligodeoxyribonucleotides derived from the translation initiation site, e.g., between the -10 and +10 regions of the target gene, are preferred. Antisense approaches involve the design of oligonucleotides (either DNA or RNA) that are complementary to mRNA encoding the target polypeptide. The antisense oligonucleotides will bind to the mRNA transcripts and prevent translation. Absolute complementarity, although preferred, is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well (Wagner, Nature 372: 333, 1994). Therefore, oligonucleotides complementary to either the 5' or 3' untranslated, non-coding regions of a gene could be used in an antisense approach to inhibit translation of that mRNA. Oligonucleotides complementary to the 5' untranslated region of the mRNA should include the complement of the AUG start codon. Antisense oligonucleotides complementary to mRNA coding regions are less efficient inhibitors of translation but could also be used in accordance with the invention. Whether designed to hybridize to the 5', 3' or coding region of mRNA, antisense nucleic acids should be at least six nucleotides in length, and are preferably less that about 100 and more preferably less than about 50, 25, 17 or 10 nucleotides in length.

It is preferred *that in vitro* studies are first performed to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Results obtained using the antisense oligonucleotide may be compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The antisense oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors), or agents facilitating transport across the cell membrane (see, e.g., Letsinger et al., Proc. Natl. Acad. Sci. U.S.A. 86: 6553-6556, 1989; Lemaitre et al., Proc. Natl. Acad. Sci. 84: 648-652, 1987; PCT Publication No. WO88/09810) or the blood-brain barrier (see, e.g., PCT Publication No. WO89/10134), hybridization-triggered cleavage agents (see, e.g., Krol et al., BioTechniques 6: 958-976, 1988) or intercalating agents (see, e.g., Zon, Pharm. Res. 5: 539-549, 1988). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytiethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose. The antisense oligonucleotide can also contain a neutral peptide-like backbone. Such molecules are termned peptide nucleic acid (PNA)-oligomers and are described, e.g., in Perry-O'Keefe et al., Proc. Natl. Acad. Sci. U.S.A. 93: 14670, 1996, and in Eglom et al., Nature 365: 566, 1993. One advantage of PNA oligomers is their capability to bind to complementary DNA essentially independently from the ionic strength of the medium due to the neutral backbone of the DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet a further embodiment, the antisense oligonucleotide is an alpha-anomeric oligonucleotide. An alpha-anomeric oligonucleotide forms specific double-stranded hybrids with complementary RNA in which, contrary to the usual antiparallel orientation, the strands run parallel to each other (Gautier et al., Nucl. Acids Res. 15: 6625-6641, 1987). The oligonucleotide is a 2'-0-methylribonucleotide (Inoue et al., Nucl. Acids Res. 15: 6131-6148, 1987), or a chimeric RNA-DNA analogue (Inoue et al., FEBS Lett. 215: 327-330, 1987).

While antisense nucleotides complementary to the coding region of a target mRNA sequence can be used, those complementary to the transcribed untranslated region may also be used.

In certain instances, it may be difficult to achieve intracellular concentrations of the antisense sufficient to suppress translation on endogenous mRNAs. Therefore a preferred approach utilizes a recombinant DNA construct in which the antisense oligonucleotide is placed under the control of a strong pol III or pol II promoter. The use of such a construct to transfect target cells will result in the transcription of sufficient amounts of single stranded RNAs that will form complementary base pairs with the endogenous potential drug target transcripts and thereby prevent translation. For example, a vector can be introduced such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, Nature 290: 304-310, 1981), the promoter contained in the 3' long terminal repeat of Rous sarcoma virus (Yamamoto et al., Cell 22: 787-797, 1980), the herpes thymidine kinase promoter (Wagner et al., Proc. Natl. Acad. Sci. U.S.A. 78: 1441-1445, 1981), the regulatory sequences of the metallothionein gene (Brinster et al, Nature 296: 39-42, 1982), etc. Any type of plasmid, cosmid, YAC or viral vector can be used to prepare the recombinant DNA construct, which can be introduced directly into the tissue site.

Alternatively, target gene expression can be reduced by targeting deoxyribonucleotide sequences complementary to the regulatory region of the gene (i.e., the promoter and/or enhancers) to form triple helical structures that prevent transcription of the gene in target cells in the body (see generally, Helene, Anticancer Drug Des. 6(6): 569-84, 1991; Helene et al., Ann. N.Y. Acad. Sci., 660: 27-36, 1992; and Maher, Bioassays 14(12): 807-15, 1992).

Nucleic acid molecules to be used in triple helix formation for the inhibition of transcription are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential target sequences that can be targeted for triple helix formation may be increased by creating a so called "switchback" nucleic acid molecule. Switchback molecules are synthesized in an alternating 5'-3',3'-5' manner, such that they base pair with first one strand of a duplex and then the other, eliminating the necessity for a sizable stretch of either purines or pyrimidines to be present on one strand of a duplex.

In certain embodiments, the antisense oligonucleotides are morpholino antisenses. Morpholinos are synthetic molecules which are the product of a redesign of natural nucleic acid structure. Usually 25 bases in length, they bind to complementary sequences of RNA by standard nucleic acid base-pairing. Structurally, the difference between morpholinos and DNA is that while morpholinos have standard nucleic acid bases, those bases are bound to morpholine rings instead of deoxyribose rings, and linked through phosphorodiamidate groups instead of phosphates. Replacement of anionic phosphates with the uncharged phosphorodiamidate groups eliminates ionization in the usual physiological pH range, so morpholinos in organisms or cells are uncharged molecules. Morpholinos are not chimeric oligos; the entire backbone of a morpholino is made from these modified subunits. Morpholinos are most commonly used as single-stranded oligos, though heteroduplexes of a morpholino strand and a complementary DNA strand may be used in combination with cationic cytosolic delivery reagents.

Unlike many antisense structural types (e.g., phosphorothioates), morpholinos do not degrade their target RNA molecules. Instead, morpholinos act by "steric blocking," binding to a target sequence within an RNA and simply getting in the way of molecules which might otherwise interact with the RNA. Morpholino oligos are often used to investigate the role of a specific mRNA transcript in an embryo, such as eggs or embryos of zebrafish, African clawed frog (Xenopus), chick, and sea urchin, producing morphant embryos. With appropriate cytosolic delivery systems, morpholinos are effective in cell culture.

Bound to the 5'-untranslated region of messenger RNA (mRNA), morpholinos can interfere with progression of the ribosomal initiation complex from the 5' cap to the start codon. This prevents translation of the coding region of the targeted transcript (called "knocking down" gene expression). Morpholinos provide a convenient means of knocking down expression of the protein and learning how that knockdown changes the cells or organism. Some morpholinos knock down expression so effectively that after degradation of preexisting proteins the targeted proteins become undetectable by Western blot.

Morpholinos can also interfere with pre-mRNA processing steps, usually by preventing the splice-directing snRNP complexes from binding to their targets at the borders of introns on a strand of pre-RNA. Preventing U1 (at the donor site) or U2/U5 (at the polypyrimidine moiety and acceptor site) from binding can cause modified splicing, commonly leading to exclusions of exons from the mature mRNA. Targeting some splice targets results in intron inclusions, while activation of cryptic splice sites can lead to partial inclusions or exclusions. Targets of U11/U12 snRNPs can also be blocked. Splice modification can be conveniently assayed by reverse-transcriptase polymerase chain reaction (RT-PCR) and is seen as a band shift after gel electrophoresis of RT-PCR products.

Morpholinos have also been used to block miRNA activity, ribozyme activity, intronic splice silencers, and splice enhancers. U2 and U12 snRNP functions have been inhibited by Morpholinos. Morpholinos targeted to "slippery" mRNA sequences within protein coding regions can induce translational frameshifts. Activities of Morpholinos against this variety of targets suggest that Morpholinos can be used as a general-purpose tool for blocking interactions of proteins or nucleic acids with mRNA.

In a particular embodiment the pannexin inhibitor is selected from the compounds of Table I, including combinations thereof In a preferred embodiment the pannexin inhibitor is selected from the group consisting of carbenoxolone (CBX), mefloquine (MFQ), flufenamic acid (FFA) and combinations thereof.

Effective amounts of the pannexin hemichannel inhibitor will depend upon the mode of administration, frequency of administration, nature of the treatment, age and condition of the individual to be treated, and the type of pharmaceutical composition used to deliver the compound into a living system. Effective levels of pannexin hemichannel blocker may range from 50 nM to 5 µM (given to experimental animals as 20-30 mg/kg twice daily for ten days), depending upon the compound, system, experimental and clinical endpoints, and toxicity thresholds. While individual doses vary, optimal ranges of effective amounts may be determined by one of ordinary skill in the art. For pannexin hemichannel inhibitors that are involved in clinical trials for other indications, the safe and effective dosages identified in such trials can be considered when selecting dosages for treatments according to the present invention.

The pannexin hemichannel inhibitor used according to the methods of the present invention can be administered alone or as a pharmaceutical composition, which includes the compound(s) and a pharmaceutically-acceptable carrier. The pannexin hemichannels blockers are typically provided as a pharmaceutical composition. The pharmaceutical composition can also include suitable excipients, or stabilizers, and can be in solid or liquid form such as, tablets, capsules, powders, solutions, suspensions, or emulsions. Typically, the composition will contain from about 0.01 to 99 percent, preferably from about 5 to 95 percent of active compound(s), together with the carrier.

The pannexin hemichannel inhibitor, when combined with pharmaceutically or physiologically acceptable carriers, excipients, or stabilizers, whether in solid or liquid form such as, tablets, capsules, powders, solutions, suspensions, or emulsions, can be administered orally, parenterally, subcutaneously, intravenously, intramuscularly, intraperitoneally, by intranasal instillation, by implantation, by intracavitary or intravesical instillation, intraocularly, intraarterially, intralesionally, transdermally, or by application to mucous membranes, such as, that of the nose, throat, and bronchial tubes (i.e., inhalation).

For most therapeutic purposes, the pannexin hemichannel inhibitor can be administered orally as a solid or as a solution or suspension in liquid form, via injection as a solution or suspension in liquid form, or via inhalation of a nebulized solution or suspension. The solid unit dosage forms can be of the conventional type. The solid form can be a capsule, such as an ordinary gelatin type containing the compounds of the present invention and a carrier, for example, lubricants and inert fillers such as, lactose, sucrose, or cornstarch. In another embodiment, these compounds are tableted with conventional tablet bases such as lactose, sucrose, or cornstarch in combination with binders like acacia, cornstarch, or gelatin, disintegrating agents, such as cornstarch, potato starch, or alginic acid, and a lubricant, like stearic acid or magnesium stearate.

For injectable dosages, solutions or suspensions of these materials can be prepared in a physiologically acceptable diluent with a pharmaceutical carrier. Such carriers include sterile liquids, such as water and oils, with or without the addition of a surfactant and other pharmaceutically and physiologically acceptable carrier, including adjuvants, excipients or stabilizers. Illustrative oils are those of petroleum, animal, vegetable, or synthetic origin, for example, peanut oil, soybean oil, or mineral oil. In general, water, saline, aqueous dextrose, and related sugar solution, and glycols, such as propylene glycol or polyethylene glycol, are preferred liquid carriers, particularly for injectable solutions.

For use as aerosols, the compound in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

For transdermal routes, the compound is present in a carrier which forms a composition in the form of a cream, lotion, solution, and/or emulsion. The composition can be included in a transdermal patch of the matrix or reservoir type as are conventional in the art for this purpose.

It is contemplated the use of combinations of the pannexin hemichannel inhibitors described in Table I.

It is also contemplated that administration of the pannexin hemichannel inhibitor can be carried out in combination with other suitable therapeutic treatments which are useful for treating CNS ischemia, thus, in other embodiment, the pannexin inhibitor can be combined with other drugs for the treatment of ischemia such as nitrates, beta-blockers (e.g., atenolol (Tenormin), metoprolol (Lopressor), nadolol (Corgard), propranolol (Inderal), timolol (Blocadren), etc.), and calcium channel blockers (e.g., amlopidine (Norvasc), diltiazem (Cardizem), isradipine (DynaCirc), nifedipine (Adalat, Procardia), nicardipine (Cardene), vera-pamil (Calan, Isoptin, Verelan), acetylsalicylic acid (Aspirin), etc.).

In a preferred embodiment the drug for the treatment of ischemia is a P2X inhibitor.

The surface receptors for extracellular nucleotides are called P2 receptors. Previously, they were called P2 purinoreceptors, but it is now realized that some P2 receptors are activated by both pyrimidine and purine nucleotides. Thus, the nomenclature has changed to reflect the varied nature of ligands for P2 receptors. The current nomenclature system for P2 receptors is based on molecular structure and signal transduction mechanisms to define families of ionotropic P2X receptors (ligand-gated ion channels) and metabotropic P2Y receptors (G protein-coupled receptors).

"P2X channels" or "P2X receptors" are ATP-gated cation channels that mediate fast excitatory transmission in diverse regions of the brain and in spinal cord (North, Physiol Rev 82:1013-1067 (2002), which is hereby incorporated by reference in its entirety). Several P2X receptor subtypes have the unusual property of changing their ion selectivity during prolonged exposure to ATP. Brief exposure to ATP induces opening of channels permeable to Na⁺ and K⁺, whereas longer exposures of seconds to minutes duration result in progressive dilatation of the channel pore, and permeability to larger cations, as well as to fluorescent indicators such as propidium, YO-PRO-1, and ethidium (Khakh et al., Nat Neurosci 2:322-330, 1999), which is hereby incorporated by reference in its entirety). The P2X7 receptor was originally described in cells of hematopoietic origin, among which its activation has been linked to cell lysis, an apparent consequence of efflux of essential metabolites and intracellular messengers (Di Virgilio et al., Cell Death Differ 5:191-199, 1998), which is hereby incorporated by reference in its entirety). The P2X7 receptor has been reported in the nervous system.

Members of the existing family of ionotropic P2X1-7 receptors show a subunit topology of i) intracellular N- and C-termini possessing consensus binding motifs for protein kinases, ii) two transmembrane spanning regions (TM1 and TM2), the first involved with channel gating and second lining the ion pore, iii) large extracellular loop, with 10 conserved cysteine residues forming a series of disulphide bridges, iv) hydrophobic H5 region close to the pore vestibule, for possible receptor/channel modulation by cations (magnesium, calcium, zinc, copper and protons ions), and v) an ATP-binding site, which may involve regions of the extracellular loop adjacent to TM1 and TM2. The P2X1-7 receptors show 30-50% sequence identity at the peptide level. The stoichiometry of P2X1-7 receptors is now thought to involve three subunits which form a stretched trimer. See Barnard et al., (Molecular Neurobiology pp. 103-129 1997), which is hereby incorporated by reference in its entirety.

P2X1-5,7 receptors can form homomultimeric assemblies by using the same subunits, although in some tissues, P2X receptors exist as heteromultimeric assemblies (P2X2/3 in nodose ganglia, P2X2/6 and P2X4/6 in CNS neurons, P2X1/5 in some blood vessels). The P2X receptor family shows many pharmacological operational differences. Agonist potency orders can vary significantly between P2X subtypes, and some (P2X4 and P2X4/6) are relatively insensitive to known P2 receptor antagonists. The kinetics of activation, inactivation, and deactivation also vary considerably amongst P2X receptors. Calcium permeability is high for some P2X subtypes, a property that may be functionally important. The P2X7 subtype converts to a pore and, in some cases, brings about cell death. Several other P2X receptors (P2X2, P2X2/3, P2X4 and P2X2/6) also show time-dependent changes in their ion permeability properties of their intrinsic ion channel. See Barnard et al., (1997. Molecular Neurobiology pp. 103-129)

A "P2X inhibitor", as used herein, relates to any compound capable of causing a decrease in the P2X activity, including those compounds which prevent expression of the P2X gene, leading to reduced P2X mRNA or protein levels as well as compounds that inhibit P2X causing a decrease in the activity of the channel like an antagonist. In a particular embodiment, the P2X inhibitor is a P2X antagonist.

An "antagonist" is a type of receptor ligand or drug that does not provoke a biological response itself upon binding to a receptor, but blocks or dampens agonist-mediated responses. A "P2X antagonist", as used herein, relates to any compound capable of binding to the P2X receptor that block the binding of ATP to the P2X receptor and thus interferes with the ion transmission through the channel.

Although P2X7 are particularly useful in carrying out the present invention, P2X1, P2X2, P2X3, P2X4, P2X5, and P2X6 purine receptor antagonists can also be utilized.

Table II include illustrative, non-limitative, P2X inhibitors.

**Table II**

| **P2X inhibitors** | |
|---|---|
| | **Wide spectrum P2X antagonist** |
| I | 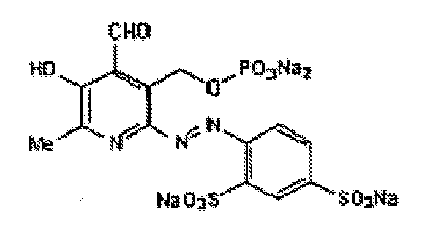 |
| | PPADS (tetrasodium salt of pyridoxalphosphate-6-azophenyl-2',4'-disulfonic acid) |
| II | 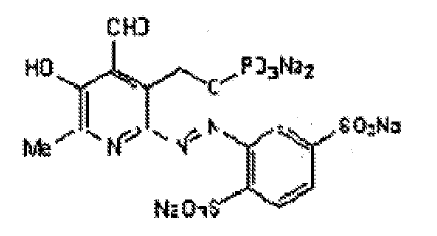 |
| | iso-PPADS (tetrasodium salt of pyridoxalphosphate-6-azophenyl-1',4'-disulfonic acid) |
| III | 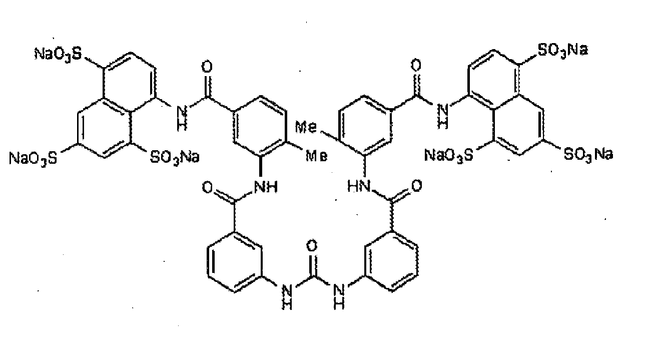 |
| | Suramin (hexasodium salt of 8,8'-[carbonylbis[imino-3,1-phenylenecarbonylimino(4-methyl-3,1-phenylene)carbonylimino]] bisnaphthalene-1,3,5-trisulphonic acid |
| IV | 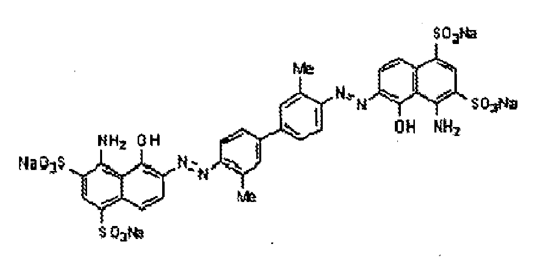 |
| | Evans Blue (tetrasodium salt of 6,6'-[(3,3'-dimethyl[1,1'-biphenyl]-4,4'-diyl)bis[4-amino-5-hydroxy-1,3-naphthalenedisulfonic acid]) |
| V | 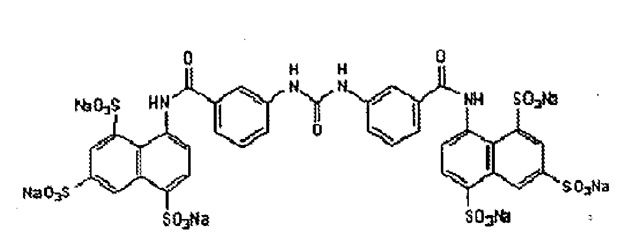 |
| | NF023 (hexasodium salt of 8,8'-[carbonylbis[imino-3,1-phenylenecarbonylimino]bis-1,3,5-naphthalenetrisulfonic acid] |
| VI | 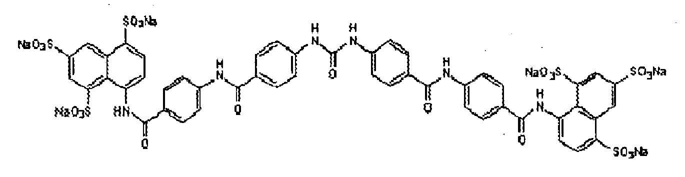 |
| | NF279 Hexasodium salt of (8,8'-[carbonylbis(imino-4,1-phenylenecarbonylimino-4,1-phenylenecarbonyl-imino))bis(1,3,5-naphthalenetrisulfonic acid] |
| VIII | 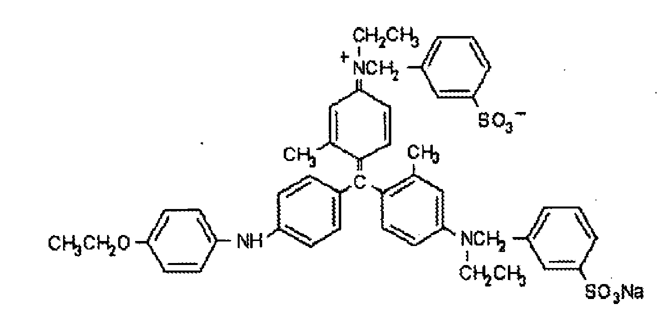 |
| | CBB-G (Coomassie brilliant blue G or Brillant Blue G) |
| IX | 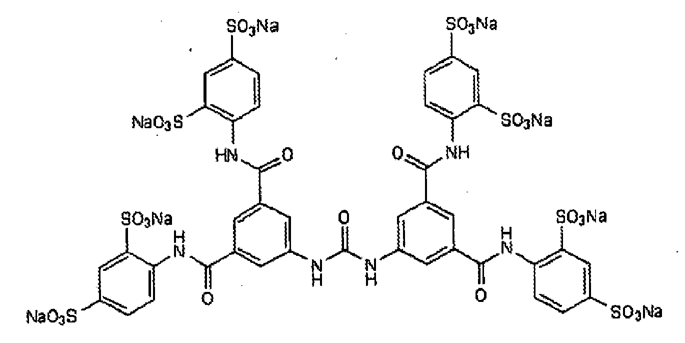 |
| | NF449 (octasodium salt of 4,4',4",4"'-(carbonylbis(imino-5,1,3-benzenetriylbis(carbonylimino)))tetrakis-benzene-1,3-disulfonic acid) |
| | **Selective P2X receptor antagonist** |
| X | 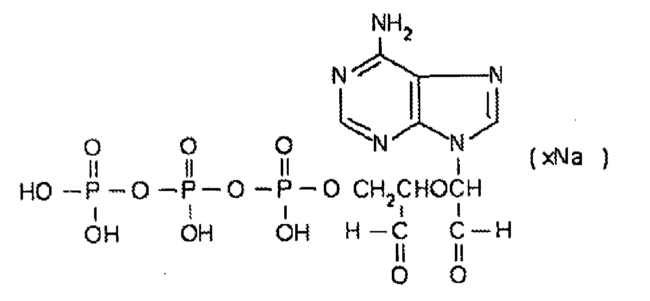 |
| | o-ATP: the sodium salt of adenosine 5-triphosphate, oxidized with periodate |
| XI | 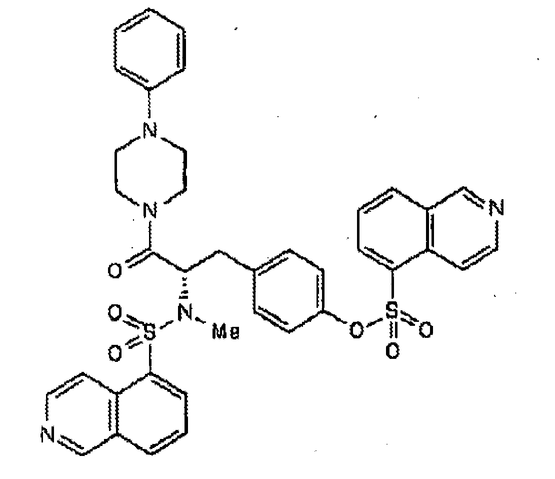 |
| | KN-62 (ester of 4-[(2S)-2-[(5-isoquinolinylsulfonyl)methylamino]-3-oxo-3-(4-phenyl-1-piperazinyl)propyl] phenyl isoquinolinesulfonic acid) |
| XII | 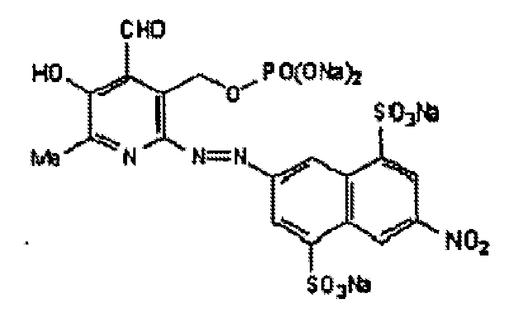 |
| | PPNDS (tetrasodium salt of pyridoxal-5'-phosphate-6-(2'-naphthylazo-6'-nitro-4', 8'-disulfonate) |
| XIII | 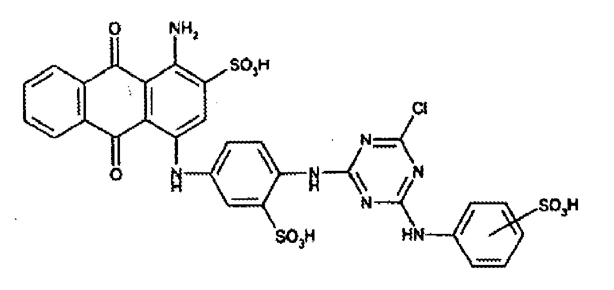 |
| | RB2 (1-amino-4-[[4-[[4-chloro-6-[[3 (or 4)-sulfophenyl]amino]-1,3,5-triazin-2-yl]amino]-3-sulfophenyl]amino]-9,10-dihydro-9,10-dioxo-2-anthracenesulfonic acid) |
| | **Other Selective P2X receptor inhibitors** |
| XIV | An inhibitory antibody capable of specifically inhibiting the activity of a P2X receptor. |
| XV | A ribozyme or a DNA enzyme specific for the sequence of a P2X receptor. |
| XVI | An interfering RNA specific for the sequence of a P2X receptor. |
| XVII | An inhibiting peptide specific for a P2X receptor. |
| XVIII | A P2X receptor specific antisense nucleic acid. I |

In a particular embodiment, the P2X inhibitor is an inhibitory antibody, a ribozyme, an interfering RNA, an inhibiting peptides specific for a P2X channel or a P2X receptor specific antisense nucleic acid.

The definition, design strategy, synthesis, etc of inhibitory antibodies, ribozyme, interfering RNA, inhibiting peptides specific for a P2X channel specific antisense nucleic acid is essentially the same as it was described previously for pannexin channels.

Besides those mentioned previously there are other wide spectrum antagonists of P2X channels such as MRS2220 (cyclic pyridoxine-alpha.4,5-monophosphate-6-azo-phenyl-2',5'disulfonate), Ip51 (pentapotassium salt of P.1,P.5-Diinosine-5-pentaphosphate) or TNP-ATP (monolithium salt of 2',3'-O-2,4,6-trinitrophenyl-adenosine 5'-triphosphate), as well as selective ones such as, for example, HMA (5-(N,N hexamethylene amiloride), or o-ATP. The IC₅₀ of some of the previous compounds in relation to the different PX2 receptor subgroups are set out in Table III.

**Table III**

| **IC50 of P2X antagonist in relation to each P2X receptor subtype** | | | | | | | |
|---|---|---|---|---|---|---|---|
| P2X subtypes | P2X1 | P2X2 | P2X3 | P2X4 | P2X5 | P2X6 | P2X7 |
| | | | | | | | |
| Antagonists: IC50 (µM) | PPADS: 1-5 | PPADS: 2 | PPADS: 1 | PPADS: 27.5 | PPADS: 2.6 | PPADS., >100 | PPADS: 4.2 |
| | Suramin: 1-5 | Suramin: 1-5 | Suramin: 3 | Suramin, 178 | Suramin: 4 | Suramin: >100 | Suramin: 4 |
| | NF023: 0.21 | NF 023: 63 | NF 023< 29 | NF 023 : >100 | | | |
| | NF279: 002 | NF279; 0.77 | NF279:1.6 | NF279: >30 | | | NF279: 2.8 |
| | | | | | | | KN-62: 0.015 |
| | Evans Blue: 1400 | Evans Blue: 1-400 | Evans Blue: 1400 | Evans Blue: 1-400 | Evans Blue: 1-400 | Evans Blue: 1-400 | Evans Blue: 1-400 |
| | isoPPADS: 1-5 | | isoPPADS: 1 | | | | |
| | | RB-2: 1 | | | | | |
| | | | | | | | HMA: 4,5 |
| | | | | | | | o-ATP 5 |
| | | | BSG: >10 | | | | BBG: 0.01 |
| | Ip51; 0.003 | | IPSk 3 | | | | |
| | MRS2220: 10 | | MRS2220: 58 | | | | |
| | NF449: 0.01 | | NF449: <0.006 | | | | |
| | PPNDS: | 0.015 | | | | | |
| | TNP- ATP: 0.001 | TNP- ATP: 1 | TNP- ATP: 0.001 | TNP- ATP: 15 | | | TNP-ATP: >30 |

Suitable P2X1 receptor antagonists include pyridoxal-5'-phosphate-6-azophenyl-2',5'-disulphonic acid, diinosine pentaphosphate, pyridoxal-5'-phosphate-6-azophenyl-4'-carboxylate, 8,8'-(carbonylbis(imino-3,1-phenylenecarbonylimino)bis(1,3,5-napththalenetrisulfonic acid), 8,8'-(carbonylbis(imino-4,1-phenylenecarbonylimino-4, 1-phenylenecarbonylimino)bis(1,3,5-napththalenetrisulfonic acid), pyridoxal-5'-phosphate-6-(2'-naphthylazo-6-nitro-4',8'-disulphonate), Suramin, and 2',3'-O-(2,4,6-trinitrophenyl)adensine triphosphate.

Useful P2X2 receptor antagonists include Reactive blue 2 and Suramin.

Suitable P2X3 receptor antagonists are pyridoxal-5'-phosphate-6-azo- phenyl-2',5'-disulphonic acid, diinosine pentaphosphate, 8,8'-(carbonylbis(imino-3,1-phenylenecarbonylimino)bis(1,3,5-napththalene- trisulfonic acid), Suramin, and 2',3'-O-(2,4,6-trinitrophenyl)adenosine triphosphate.

Brilliant blue G can be utilized as a P2X4 receptor antagonist, while Suramin is a suitable P2X5 receptor antagonist.

In a preferred embodiment the P2X inhibitor is a P2X7 inhibitor. In a more preferred embodiment the P2X7 inhibitor is selected from the group consisting of O-ATP, Coomassie Brilliant Blue G or PPADS.

In another preferred embodiment the P2X inhibitor is selected from the group consisting of o-ATP, Coomassie Brilliant Blue G or PPADS.

Another aspect of the invention refers to a pharmaceutical composition which comprises of at least one P2X purinergic receptor antagonist, either wide spectrum or selective of a receptor subgroup, along with at least one pharmaceutically accepted excipient. Accepted excipients and ways of administration have been previously described.

A further aspect of the present invention relates to a method of treating a subject with ischemic or traumatic insults of brain or spinal cord tissue in regions expressing pannexin hemichannels. This method involves administering a pannexin hemichannel blocker to the subject under conditions effective to treat ischemic or traumatic insults of brainor spinal cord tissues in regions expressing pannexin hemichannels. The pannexin hemichannels inhibits the ATP flow through the pannexin hemichannels.

This aspect of the present invention is carried out in substantially the same manner as described above.

Another aspect of the present invention relates to a method of inhibiting ATP-triggered central nervous system death, said method comprising contacting central nervous system cells with a pannexin inhibitor under conditions effective to inhibit ATPtriggered central nervous system cell death, wherein the inhibitor inhibits the ATP flow through the pannexin hemichannel.

Causes of such central nervous system cell death include ischemia, vascular insufficiency, stroke, and traumatic brain injury. In a particular embodiment, this method can be used with brain or spinal cord cells which are neural cells (e.g., neurons, oligodendrocytes, or astrocytes) as well as with endothelial cells. Preferably, all of these cell types are from humans. In this case, the pannexin inhibitor inhibits the release of ATP through the pannexin hemichannel. In a preferred embodiment, the cells are oligodendrocytes.

In a particular embodiment, this method additionally comprises the contacting of a CNS cell with a P2X receptor inhibitor under conditions effective to inhibit ATP-triggered brain or spinal cord cell death. This method can be also used with brain or spinal cord cells which are neural cells (e.g., neurons, oligodendrocytes, or astrocytes) as well as with endothelial cells. Preferably, all of these cell types are from humans. In this case, the purine receptor inhibitor inhibits the ion flow through the P2X channel.

Pannexin inhibitor and P2X purine receptor inhibitors have been described previously.

Another aspect of the present invention relates to a P2X inhibitor for the use in the treatment of central nervous system ischemia. In a preferred embodiment, the ischemia is a white matter ischemia.

The terms "CNS ischemia", "white matter ischemia" and "P2X inhibitor" has been previously defined.

In a preferred embodiment, the P2X receptor is localized on the oligodendrocytes. In a preferred embodiment, the P2X receptor is a P2X7 receptor.

P2X receptor inhibitors useful for the present invention have been described previously.

In a particular embodiment, the P2X antagonist that can be used for the present invention are selected from between PPADS, iso-PPADS, Suramin, o-ATP, Evans Blue, NF023, NF279, CBB-G, KN-62, PPNDS, RB2, MRS2220, Ip51, TNP-ATP, HMA, a P2X inhibitory antibody, a P2X specific ribozyme or ribozyme, an interfering RNA specific for P2X receptor, an inhibiting peptides specific for a P2X channel or a P2X receptor specific antisense nucleic acid.

In a preferred embodiment the P2X inhibitor is a P2X7 inhibitor. In a more preferred embodiment the P2X7 inhibitor is selected from the group consisting of o-ATP, Coomassie Brilliant Blue G or PPADS.

### EXAMPLE 1

### Protective effect of pannexin hemichannel inhibitors and P2X7 antagonists in white matter ischemia models

### 1.1 Materials and methods

### Oxygen and glucose deprivation in oligodendrocyte cultures

Primary cultures of oligodendrocytes derived from optic nerves of 12 day-old Sprague-Dawley rats were obtained as previously described (Matute et al., 1997 Proc Natl Acad Sci U S A 94:8830-8835.).Cells were maintained at 37°C and 5% CO₂ in a chemically defined medium (Matute et al., 1997 Proc Natl Acad Sci U S A 94:8830-8835.). After 2-4 *days in vitro,* cultures were composed of at least 98% 04/Ga1C⁺ cells. No microglial cells were detected in these cultures. *In vitro* ischaemia (1h) was achieved by replacing O₂ with N₂ and external glucose (10 mM) with sucrose and adding iodoacetate (20 µM) to block glycolysis in an extracellular solution containing (in mM) NaCl (130), KCI (5.4), CaC1₂ (1.8), NaHCOₛ (26), MgC1₂ (0.8) and NaH₂PO₄ (1.18) (pH 7.4). Cell death was determined 24h later using calcein-AM (Invitrogen) as previously described (Matute et al., 1997 Proc Natl Acad Sci U S A 94:8830-8835.). Antagonists were present *during in vitro* ischaemia and during the next 24h incubation period. The total calcein fluorescence on each coverslip was measured using a Synergy-HT fluorimeter (BioTek Instruments) and data were expressed as percentage of cell death versus the respective control with or without antagonists, which did not alter cell survival (data not shown). To assay the levels of reactive oxygen species (ROS), cells were loaded with 40 µM 2',7'- dichlorodihyfluoresceindiacetate (DCFDA; Invitrogen) and 1 µM calcein red-orange (Molecular Probes) immediately after OGD. Calcein was used to quantify the number of cells within the reading field. Fluorescence was measured as above and values were plotted as the percentage of DCFDA/calcein red-orange fluorescence versus control. The changes in mitochondrial membrane potential were monitored with 5,5',6,6'-tetrachloro-1,1',3,3'-tetraethylbenzimidazolcarbocyanine iodide (JC-1; Molecular Probes). Immediately after OGD, cells were loaded with JC-1 (3 µM) at 37°C for 30 min. The changes in the membrane potential were calculated as the red (emission at 590)/green (emission at 520) ratio in each condition and results were normalized to control.

### Electrophysiology and Ca²⁺ imaging

Whole cell recordings were performed at room temperature and at a holding membrane potential of -70 mV. Extracellular bath solution with a pH of 7.3 contained (in mM): NaC1 140, KC1 5.4, CaC1₂ 2, MgC1₂ 1, HEPES 10. Patch clamp pipettes (3-5 MΩ) were filled with internal solution at a pH of 7.3 containing (in mM): CsCI 140, CaC1₂ 2, MgC1₂ 2, HEPES 10, EGTA 11, Na-ATP 2. For electrophysiology monitoring, ischaemia was induced chemically by using the glycolytic blocker iodoacetate (1 mM) and the oxidative phosphorylation inhibitor antimycin (2.5 µM), and omitting glucose because recordings were performed in an open chamber where O₂ can diffuse to the cells (Káradóttir et al., 2005). Recordings of ATP currents were performed in divalent cation-free extracellular solutions, obtained by omitting Ca²⁺ and Mg²⁺, to potentiate P2X-mediated responses (Matute et al., 1997 Proc Natl Acad Sci U S A 94:8830-8835.). For Ca²⁺ recording, cells were loaded with fura-2M (5 µM; Molecular Probes, Inc., Eugene, OR) in culture medium for 30 min at 37 °C. Experiments were carried out in a coverslip chamber continuously perfused with a buffer containing 20 mM HEPES, 2 mM CaC1₂, 10 mM glucose in HBSS at 1 ml/min. The perfusion chamber was mounted on the stage of a Zeiss inverted epifluorescence microscope (Axiovert 35), equipped with a 150 W xenon lamp Polychrome IV (TILL Photonics GMBH) and a Plan Neofluar 40x oil immersion objective (Zeiss, Germany). Cells were visualized with a high resolution digital B/W CCD camera (ORCA; Hamamatsu Photonics Iberica, Barcelona, Spain) and image acquisition and data analysis were carried out using the AquaCosmos software program (Hamamatsu Photonics Iberica). Images were acquired every 30 ms at 37°C and ischaemia was induced as above. [Ca²⁺]¡ was estimated by the 340/380 ratio method, using a Kd value of 224 nM. At the end of the assay, in situ calibration was carried out with the successive addition of 10 mM ionomycin and 2M Tris/50 mM EGTA, pH 8.5. Data were analyzed with Excel and Prism software.

### Pannexin expression analysis

Immunostaining of 04 was performed in live oligodendrocytes as previously described (Mato et al., 2009 Glia 57:295-306) using a mouse monoclonal antibody to 04 generously provided by Dr. C. Thomson (1:10, hybridoma supernatant). Oligodendroglial expression of pannexins was examined by conventional immunocytochemistry as previously described (Matute et al., 2007) using primary antibodies to pannexin 1 (1:500; Chemicon) and 2 (1 µg/ml; Abcam). Antibody labelling was visualized using secondary antibodies Texas Red-conjugated goat antimouse IgM (1:100; Calbiochem) and Alexa Fluor 488-conjugated goat anti-rabbit (1:200; Invitrogen). Controls were performed by omitting the primary antibody, which yield no labelling (data not shown).

Total RNA from cell cultures and rat tissue samples was extracted using TRIzol reagent according to the manufacturer's instructions (Invitrogen). Synthesis of cDNA was carried out with the reverse transcriptase Superscript™ III (Invitrogen) using random hexamers as primers. The reaction mixture was incubated at 50°C for 60 minutes followed by 15 min at 70°C to inactivate reverse transcriptase. Mock reverse transcription (without enzyme) for each sample served as negative controls. Primers specific for pannexin 1, 2 and 3 were designed with PrimerExpress software (Applied Biosystems, Madrid, Spain) in exon junctions to avoid amplification of any contaminating genomic DNA and are as follows:
Panx 1 forward: TGAGAATGTGGGGCAGAGTC (SEQ ID NO: 15)
Panx1 reverse: CAGTACCACCGCAAAGGTCAC (SEQ ID NO: 16)
Panx2 forward: TTCACCAAGAACTTCGCAGAG (SEQ ID NO: 17)
Panx2 reverse: CAGTAGCCACGGGCGTACA (SEQ ID NO: 18)
Panx3 forward: GTCAAGTTCGTTACTGTGGGCTT (SEQ ID NO: 19)
Panx3 reverse: AGCAGCTGATCGGAGACCCT (SEQ ID NO: 20)

Real time quantitative PCR reactions were performed in an ABI Prism 7000 Sequence Detection System Instrument (Applied Biosystems) as previously described (Vallejo-Illarramendi et al., 2006. Neurobiol Dis 21:154-164).

### ATP release measurement

ATP levels were measured using a Luciferin/Luciferase assay kit (Invitrogen) after 1h normoxia or OGD in oligodendrocyte cultures and in isolated optic nerves according to the manufacturer's instructions. The luminescence was measured with a luminometer Synergy-HT (BioTek) with an integration time of 3 sec. Because ATP was measured in the culture medium and in the solution bathing optic nerves, the concentration of ATP at the extracellular surface, near the vicinity of the receptor was underestimated (Joseph et al., 2003. J Biol Chem 278:23331-23342). The limit of ATP detection was 1 nM and luminescence was linear with increasing ATP concentrations to 1000 nM. The total amount of ATP was calculated according to standard curves and ATP release was expressed in percentage *vs* control.

### Optic nerve ischaemia and electron microcopy

Optic nerves from P28 rats (unless otherwise indicated) and P28 PLP-DsRed mice were dissected in oxygen-saturated artificial CSF (aCSF) containing (in mM); 126 NaCl, 3 KCI, 2 MgS0₄, 26 NaHC0₃, 1.25 NaH₂P0₄, 2 CaCl₂ 2H₂O, as previously described (20). To stimulate ischaemia, we replaced external O₂ with N₂ and external glucose with sucrose (10 mM) and added 1 mM iodoacetate to block glycolysis. After 1h OGD at 37°C, the medium was replaced with fresh solution containing glucose and equilibrated with 95% O₂, 5% CO₂ and the nerves were incubated for another 2h at 37°C to simulate reperfusion. Tissue damage was analyzed by measuring spectrophotometrically (*λ*= 490 nm) LDH release in the incubation medium. Data was normalized to LDH release by control nerves with or without P2X7 antagonists or hemichannels blockers respectively, all of which did not induce any damage to optic nerves (data not shown).

To ensure that LDH release was due to oligodendroglial damage, experiments were performed in transgenic mice carrying DsRed sequence under the control of mouse PLP promoter. After OGD, nerves were fixed in 4% p-formaldehyde and freeze-sectioned (10 µm). Damage was determined by imaging and Hoechst labelling. Sections were incubated with Hoechst 33258 (5 µg/ml; 10 min) for nuclear staining. Damage to optic nerves from PLP-DsRed mice after OGD was evaluated on an Olympus Fluoview FW500 confocal microscopy using 40X objective. Loss of DsRed-filled processes or of DsRed-filled somata was assessed as a change in mean pixel intensity per area within about 10 fields per section free of somata or processes respectively, and in three different sections per nerve. Images were taken using the same illumination parameters in a laser-scanning confocal microscope (Olympus Optical FluoView FV500). Comparisons were performed between nerves from the same animal in order to compensate for variations in DsRed expression between animals (n= 4 animals).

For light microscopy in semithin sections (1 µm thick) and electron microscopy, P28 rat optic nerves, subjected to ischemia as above, were fixed in 1.6% glutaraldehyde in cacodylate buffer and processed routinely for electron microscopy as previously described (Micu et al., 2006 Nature 439:988-992; Matute et al., 2007. J Neurosci 27:9525-9533). The number of pyknotic oligodendrocytes and myelin separations were counted in randomly selected fields (n= 10-30) from each treatment group. In the semithin preparation, oligodendrocytes, which constitute the vast majority of cell bodies in the optic nerve, were identified by their contiguity, reduced cytoplasm and the dark rim of clumped chromatin at the edge of their nuclei (Peters et al., 1991 Oxford University Press, p 273-311.).

### Compound action potential

Propagated compound action potentials (CAPs) in 1 month old C57BL6 mice were evoked using a bipolar silver electrode placed on one end of the optic nerve. Stimulus pulse (30 µs duration delivered every 15 s) strength was adjusted to evoke supramaximal stimulation (Tekkök et al., 2007. J Neurosci 21:4237-4248.). CAPs were recorded at 37°C with a suction electrode connected at the opposite end of the optic nerve and back-filled with aCSF (as described previously). Optic nerves were perfused (1 ml/min) with continuously bubbled with 95% O₂/5% CO₂ or 95%N₂ /5% CO₂ during normoxia and OGD respectively. The signal was amplified 5000X and filtered at 30 kHz. After recording control CAPs for 30 min, ischaemia was induced by combined oxygen and glucose deprivation (OGD) for 1h. CAP recovery after OGD was recorded 1h after reoxygenation and reperfusion with glucose. Ca²⁺-free aCSF was applied exclusively during OGD (200 µM EGTA + 4 mM Mg²⁺). Drugs were applied 30 min before, during and 1h after OGD. TTX (1 µM) was applied at the end of the experiment to obtain the stimulus artefact, which was subtracted from all the records.

### Statistics

Data are presented as mean ± s.e.m. P values are from Student's two tailed t-test except for multiple comparisons, which were performed with one-way analysis of variance (ANOVA) and Bonferroni post-hoc tests.

### 1.2 Results

### P2X7 antagonists suppress ischemic inward currents, Ca²⁺ increase and cell death in oligodendrocytes

To examine the role of P2X receptors in white matter ischaemia, the inventors simulated energy deprivation by blocking glycolysis and mitochondrial function with iodoacetate and antimycin, respectively, in a medium lacking glucose (replaced with sucrose). Chemical ischaemia activated an inward current in oligodendrocytes within 4.8 ± 0.8 min (Fig. 1A; peak inward current at -70 mV was 167 ± 38 pA, n= 67). Ischaemia-induced currents were generated by activation of purinergic receptors, since they were significantly reduced by pyridoxal phosphate 6-azophenyl-2,4'-disulphonic acid (PPADS) (100 µM), a non-selective antagonist of P2X receptors (Fig. 1A; n=6). Periodate oxydized ATP (oATP, 1 mM), a preferential antagonist of P2X7 receptors, and Brilliant Blue G (BBG, 50 nM), a selective antagonist of P2X7 receptors at this concentration (Anderson and Nerdergaard, 2006. J. Neurochem 88:246-256), significantly reduced the ischaemic inward currents (n= 14 and 10 respectively; Fig. 1A). The effect of BBG and PPADS were reversed after washing the antagonists out (see Fig. 1A; OGD currents after drug application were on average 134 ± 32% versus OGD currents before drug application; n= 15). Chemical ischaemia also induced a slow increase in the cytosolic concentration of [Ca²⁺]¡ in oligodendrocytes within minutes (Fig. 1B). The rise in [Ca²⁺]¡ after ischaemia is mainly attributable to Ca²⁺ influx through the plasma membrane and marginally to Ca²⁺ release from intracellular stores because it was abolished by removal of Ca²⁺ (0 Ca²⁺ + 50 µM EGTA to chelate any residual Ca²⁺) from extracellular medium (n= 24; Fig. 1B). The increase in [Ca²⁺]¡ caused by ischaemia was significantly blocked by PPADS and oATP (Fig. 1B), indicating that ischaemia activates P2X7 receptors in oligodendrocytes.

Then the effect of P2X7 activation on oligodendroglial damage after ischaemia was tested. Exposure of cultured oligodendrocytes to oxygen-glucose deprivation (OGD) induced mitochondrial membrane depolarization, an increase in the generation of reactive oxygen species (ROS) (Fig. 1C) and cell death (32.7 + 5.1% oligodendrocyte death, n= 26; Fig. 1D). Cell death was dependent on extracellular Ca²⁺ entry through ionotropic glutamate receptors and other channels since removal of Ca²⁺ from the culture medium abolished oligodendrocyte death (Fig. 1D). However, CNQX (30 µM) plus APV (100 µM) did not completely block OGD-induced oligodendrocyte cell death (Fig. 1D), suggesting that another route of Ca²⁺ entry mediates oligodendrocyte death. Indeed, oligodendrocyte damage was significantly reduced by purinergic antagonist PPADS (100 µM) and by BBG at a P2X7-selective concentration (50 nM) (Fig. 1C and D).

The effects described above are not due to non-specific interactions of P2X7 antagonists on ionotropic glutamate receptors since these drugs did alter oligodendrocyte death induced by AMPA or kainate receptor activation or NMDA-induced neuronal death (Fig. 8). Effects of P2X7 antagonists on NMDA-induced cell death were analyzed in cultured neurons because NMDA did not induce cell death in oligodendrocytes *in vitro* (data not shown). In addition, simultaneous blocking of ionotropic glutamate and P2X7 receptors further reduced OGD-induced currents (n= 12; Fig. 1A) and oligodendrocyte cell death (Fig. 1D). Moreover, BBG inhibited OGD-induced currents in oligodendrocytes in which CNQX (30 µM) plus APV (100 µM) were applied before the onset of ischemia (n= 8, Fig. 1E), indicating that both P2X7 and ionotropic glutamate receptors are activated after ischemia.

These findings suggest that oligodendrocytes release ATP in response to OGD, even during glutamate receptor blockade. Ensuing activation of the pore-forming P2X7 receptor induces mitochondrial depolarization and ROS generation culminating in oligodendrocyte death.

### Oligodendrocytes release ATP through pannexin hemichannels after ischemia

To explore this possibility more directly, the inventors employed luminescence to measure ATP in the culture medium after OGD using the luciferin/luciferase-based method. OGD induced an increase in ATP concentration (181.2 ± 25.3 %; n= 11), which was potentiated in the presence of the ecto-ATPase inhibitor ARL67156 (which prevents ATP conversion into adenosine) (100 µM; 439.6 ± 71.0 %, n= 7, Fig. 2A). In line with that finding, ARL67156 (100 µM) increased oligodendroglial vulnerability to OGD (Fig. 2B) whereas the ATP-degrading enzyme apyrase (20 U/ml) reduced oligodendroglial cell death (Fig. 2B).

Next the mechanism by which ATP is released was examined by the inventors. Pannexin 1 and 2 mRNA, but not pannexin 3, was detected in oligodendrocytes by qPCR (Fig. 2C). Immunocytochemistry analysis revealed that both pannexin 1 and 2 are located in oligodendroglial somata and processes (Fig. 2D). Moreover, the non-specific gap junction blocker carbenoxolone (CBX; 20 µM) and the pannexin hemichannel blockers mefloquine (MFQ; µM) and flufenamic acid (FFA; 100 µM) reduced ATP levels after OGD, as analyzed in the presence of ARL67156 to inhibit ATP degradation by ecto-ATPases (Joseph et al., 2003. J Biol Chem 278:23331-23342.) (Fig. 2A). Consistently, these hemichannel blockers also protected against OGD-induced oligodendrocyte death (Fig. 2B). In the experimental conditions used in this study the hemichannel blockers did not act as antagonists of P2X7 receptors as reported in other preparations (Suadicani et al., 2006 Science 272:735-738), since they did not alter ATP-evoked membrane currents (Fig. 3A) and BzATP-toxicity in oligodendrocyte cultures (Fig. 3B). Taken together, these data indicate that OGD to oligodendrocytes opens pannexin-like hemichannels and induces ATP release which kills oligodendrocytes by activation of P2X7 receptors.

### P2X7 antagonists prevent oligodendrocyte cell death, myelin damage and axon dysfunction after white matter ischemia

Inventors then analyzed the effect of ischaemia in intact adult rat optic nerves. OGD (1h) induced massive cell damage which was monitored by measuring lactate dehydrogenase (LDH) release (n= 13; Fig. 4A). In turn, imaging of mouse optic nerves expressing DsRed fluorochrome under the control of the PLP gene promoter confirmed that oligodendrocytes were severely damaged as they showed loss of fluorescence intensity in processes and in somata (Fig. 4B; mean in arbitrary units in processes from controls 5.92 ± 0.7 and after OGD 1.03 0.9, p<0.0001; in somata from controls 29.0 ± 1.4 and after OGD 16.8 ±1.1, p<0.0001) as well as nuclei condensation (arrows in Fig. 4B). Removing extracellular Ca²⁺ or incubation with P2X7 receptor antagonists PPADS (1 mM), BBG (50 nM) and oATP (1mM) reduced ischaemia-induced damage to optic nerves (Fig. 4A). The relative contribution of P2X7 receptors to OGD damage was analyzed during oligodendrocyte post-natal development using premyelinating (P7), myelinating (P14) and mature optic nerves (P28). P2X7 receptor antagonists BBG and oATP protected against OGD at all developmental stages (Fig. 4C). Detailed analysis in semithin sections demonstrated that oligodendrocytes identified on the basis of their size, shape and nuclear appearance showed signs of injury after OGD, such as pyknotic nuclei (arrows in Fig. 5). Control optic nerves with or without P2X7 antagonists (not subjected to ischaemia) were practically devoid of damaged cells (data not shown). The number of pyknotic oligodendrocytes was greatly reduced in the presence of BBG (50 nM; p<0.0001) and oATP (1 mM; p<0.0001) (Fig. 5).

Because P2X7 receptors are located mainly in oligodendrocytes, in myelin sheaths as well as in oligodendroglial somata, but not in axons (see Fig. 9) (Matute et al., 2007 J Neurosci 27:9525-9533), damage to ischaemic optic nerves using electron microscopy was examined. Myelin was focally disrupted in many axons after OGD, showing loosening and separation of lamellae (arrowheads in Fig. 5). Myelin damage was significantly reduced in the presence of BBG (50 nM; n=10; p <0.05) and oATP (1 mM; n= 22; p<0.0001) (Fig. 5). These data suggest that the extracellular concentration of ATP rises during ischaemia to a level sufficient to activate P2X7 receptors. Indeed, we detected a dramatic increase in ATP levels in the perfusate bathing the optic nerves as early as 5 min after inducing OGD, peaking at 20 min (Fig. 6A). ATP levels during OGD were reduced by pannexin hemichannel inhibitors MFQ and CBX (Fig. 6A) and accordingly, optic nerve damage induced by OGD was attenuated by the ATP-degrading enzyme apyrase and by inhibitors of pannexin hemichannel MFQ, CBX and FFA (Fig. 6B).

In myelinated white matter tracts such as the optic nerve, axon loss of excitability after ischaemia is partially mediated by glutamate excitotoxicity to oligodendrocytes (Tekkok et al., 2007 J Cereb Blood Flow Metab 27:1540-1552; Underhill and Goldberg, 2007. Neurobiol Dis 25:284-290). We therefore next tested whether ATP excitotoxicity to oligodendrocytes could mediate the loss of action potential propagation occurring after ischaemia. In control optic nerves perfused with normal artificial cerebrospinal fluid (aCSF), electrophysiological recordings of compound action potential (**CAPs**) showed no significant change after 3h. In contrast, prolonged perfusion with ATP (10 mM; 3h) provoked a reduction in CAP area of 50.1 ± 10.5% as compared to matched controls (n= 4; Fig. 6C), which did not recover after 1h wash with normal aCSF (data not shown). These results indicate that, like glutamate (Li et al., 1999), ATP excitotoxicity causes conduction failure in the optic nerve.

Finally, it was tested whether P2X7 receptor antagonist prevented loss of white matter function after OGD. In control, vehicle-treated nerves, **CAPs** completely disappeared in optic nerves electrically stimulated after 1h OGD, and partially recovered 1h later when returning to normal solution containing oxygen and glucose (Fig. 7). The loss of function detected after 1h OGD is strongly dependent on the presence of extracellular Ca²⁺ (Fig. 7A and D), as previously described (Tekkok et al., 2007. J Cereb Blood Flow Metab 27:1540-1552). Notably, the P2X7 antagonists BBG and oATP significantly improved the recovery of axon function after the ischaemic insult (Fig. 7B, C and D). Together, these observations indicate that tissue damage and functional impairment in the optic nerve as a consequence of ischemia is ameliorated by inhibiting ATP release, enhancing ATP degradation, and blocking P2X7 activation as well as pannexin hemichannel permeability.

## Claims

1. A pannexin hemichannel inhibitor for use in the treatment of central nervous system ischemia.

2. Pannexin hemichannel inhibitor according to claim 1, wherein the pannexin is selected from Panx 1, Panx2 and Panx 3.

3. Pannexin hemichannel inhibitor according to claim 1 or 2, wherein the pannexin inhibitor is selected from the compounds of Table 1.

4. Pannexin hemichannel inhibitor according to any of the preceding claims, wherein the pannexin inhibitor is selected from the group consisting of carbenoxolone, mefloquine, flufenamic acid and combinations thereof.

5. Pannexin hemichannel inhibitor according to any of the preceding claims, wherein the inhibitor is combined with an additional drug for the treatment of ischemia.

6. Pannexin hemichannel inhibitor according to claim 5, wherein said additional drug is a P2X inhibitor.

7. Pannexin hemichannel inhibitor according to claim 6, wherein the P2X inhibitor is selected from PPADS, iso-PPADS, Suramin, o-ATP, Evans Blue, NF023, NF279, CBB-G, KN-62, NF449, PPNDS, RB2, MRS2220, Ip5l, TNP-ATP, HMA, a P2X inhibitory antibody, a P2X specific ribozyme or ribozyme, an interfering RNA specific for P2X receptor, an inhibiting peptides specific for a P2X channel or a P2X receptor specific antisense nucleic acid, or combinations thereof, preferably, o-ATP, CBB-G, PPADS or combinations thereof.

8. Pannexin hemichannel inhibitor according to any of the preceding claims, wherein the pannexin inhibitor is administrated with a pharmaceutically acceptable carrier or excipient.

9. Pannexin hemichannel inhibitor according to any of the preceding claims, wherein the ischemia is a white matter ischemia.

10. A method of inhibiting ATP-triggered central nervous system cell death which comprises contacting central nervous system cells with a pannexin inhibitor under conditions effective to inhibit ATPtriggered central nervous system cell death, wherein the inhibitor inhibits the ATP flow through the pannexin hemichannel.

11. The method according to claim 10, wherein the central nervous system cells are selected from the group consisting of astrocytes, neurons, oligodendrocytes, and combinations thereof.

12. A P2X inhibitor for use in the treatment of central nervous system ischemia.

13. P2X inhibitor according to claim 12, wherein the P2X is P2X7.

14. P2X inhibitor according to claims 12 or 13, wherein the P2X inhibitor is selected from the group consisting of PPADS, iso-PPADS, Suramin, o-ATP, Evans Blue, NF023, NF279, CBB-G, KN-62, PPNDS, RB2, MRS2220, Ip51, TNP-ATP, HMA, a P2X inhibitory antibody, a P2X specific ribozyme or ribozyme, an interfering RNA specific for P2X receptor, an inhibiting peptide specific for a P2X channel, a P2X receptor specific antisense nucleic acid, and combinations thereof.

15. P2X inhibitor according to anyone of claims 12 to 14, wherein the ischemia is a white matter ischemia.
